# EUROPÄISCHE PATENTSCHRIFT

(11) **EP 2 119 717 B1**
(45) Veröffentlichungstag und Bekanntmachung des Hinweises auf die Patenterteilung: **17.01.2018**
(21) Anmeldenummer: 09158875.6
(22) Anmeldetag: 12.02.2005
(51) Int. Cl.: C07D 473/04, A61K 31/437, A61P 3/10

(54) **8-[3-amino-piperidin-1-yl]-Xanthine, deren Herstellung und deren Verwendung als DPP IV Hemmer**
8-[3-amino-piperidin-1-yl]-xanthins, their production and utilisation as DPP IV inhibitors
8-[3-amino-pipéridine-1-yl]-xanthine, leur fabrication et leur utilisation en tant que complexe DDP IV

(30) Priorität: 18.02.2004 DE 102004008112; 17.03.2004 DE 102004012921; 03.07.2004 DE 102004032263
(43) Veröffentlichungstag der Anmeldung: 18.11.2009
(62) Teilanmeldung aus: 05707354.6
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: Himmelsbach, Frank, 88441 Mittelbiberach (DE); Langkopf, Elke, 88447 Warthausen (DE); Eckhardt, Matthias, 88400 Biberach (DE); Tadayyon, Mohammad, Watford, Hertfordshire WD 17 4HU (GB); Thomas, Leo, 88400 Biberach (DE)
(74) Vertreter: Simon, Elke Anna Maria

(56) Entgegenhaltungen:
- WO-A-02/068420
- WO-A-2004/018468

## Beschreibung

In der vorliegenden Beschreibung wird Bezug genommen auf neue substituierte Xanthine der allgemeinen Formel 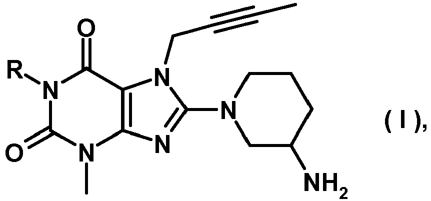 deren Tautomere, Enantiomere, Diastereomere, deren Gemische und deren Salze, insbesonders deren physiologisch verträgliche Salze mit anorganischen oder organischen Säuren, welche wertvolle pharmakologische Eigenschaften aufweisen, insbesondere eine Hemmwirkung auf die Aktivität des Enzyms Dipeptidylpeptidase-IV (DPP-IV), deren Herstellung, deren Verwendung zur Prävention oder Behandlung von Krankheiten oder Zuständen, die in Zusammenhang mit einer erhöhten DPP-IV Aktivität stehen oder die durch Reduktion der DPP-IV Aktivität verhindert oder gemildert werden können, insbesondere von Diabetes mellitus Typ I oder Typ II, die eine Verbindung der allgemeinen Formel (I) oder ein physiologisch verträgliches Salz davon enthaltenden Arzneimittel sowie Verfahren zu deren Herstellung.

Gegenstand der vorliegenden Erfindung sind die beschreibungsgemäßen obigen Verbindungen zur Verwendung gemäß den vorliegenden Ansprüchen.

Die vorliegende Erfindung betrifft ferner Arzneimittel, Verfahren sowie Verwendungen gemäß den vorliegenden Ansprüchen.

Xanthinderivate mit einer hemmenden Wirkung auf DPP-IV sind bereits aus WO 02/068420, WO 02/02560, WO 03/004496, WO 03/024965, WO 04/018468, WO 04/048379, JP 2003300977 und EP 1 338 595 bekannt.

In der obigen Formel I bedeutet
R eine Benzyl-, 2-Fluorbenzyl-, 3-Fluorbenzyl-, 4-Fluorbenzyl-, 2,6-Difluor-benzyl-, 3,4-Difluor-benzyl-, 2-Chlorbenzyl-, 3-Chlorbenzyl- oder 4-Chlorbenzyl-Gruppe,
eine 2-Trifluormethyl-benzyl-, 3-Trifluormethyl-benzyl- oder 4-Trifluormethyl-benzyl-Gruppe,
eine 3-Trifluormethoxy-benzyl- oder 4-Trifluormethoxy-benzyl-Gruppe,
eine 2-Cyanobenzyl-, 3-Cyanobenzyl- oder 4-Cyanobenzyl-Gruppe,
eine 2,6-Dicyanobenzyl-, 3,4-Dicyanobenzyl-, 3,5-Dicyanobenzyl-, 2-Trifluormethyl-4-cyano-benzyl-, 3-Nitro-4-cyano-benzyl-, 2-Cyano-3-methoxy-benzyl-, 2-Cyano-4-methoxy-benzyl-, 2-Cyano-5-methoxy-benzyl-, 2-Cyano-4-fluor-benzyl-, 2-Cyano-5-fluor-benzyl-, 2-Cyano-6-fluor-benzyl-, 3-Cyano-4-fluor-benzyl-, 4-Cyano-3-fluorbenzyl-, 2-Fluor-4-cyano-benzyl-, 2-Cyano-3-chlorbenzyl-, 2-Chlor-4-cyano-benzyl- oder 2-Cyano-4-brombenzyl-Gruppe,
eine 2-Methoxy-benzyl-, 3-Methoxy-benzyl-, 4-Methoxy-benzyl-, 2-Fluor-3-methoxy-benzyl-, 2-Fluor-4-methoxy-benzyl-, 2-Fluor-5-methoxy-benzyl-, 3-Fluor-4-methoxy-benzyl-, 3,4-Dimethoxy-benzyl-, 3,5-Dimethoxybenzyl- oder 3,4-Dimethoxy-6-fluorbenzyl-Gruppe,
eine (Benzo[1,3]dioxol-5-yl)methyl-Gruppe,
eine [(4-Cyano-benzo[1,3]dioxol-5-yl)methyl-Gruppe,
eine 2-(3-Cyclopropyloxy-phenyl)-2-oxo-ethyl-, 2-(3-Cyclopropylmethoxy-phenyl)-2-oxo-ethyl- oder 2-(3-Cyclobutyloxy-phenyl)-2-oxo-ethyl-Gruppe,
eine 2-Oxo-2-[2-(pyridin-3-yl)-phenyl]-ethyl- oder 2-Oxo-2-[2-(pyridin-4-yl)-phenyl]-ethyl-Gruppe,
eine (3-Cyano-naphthalin-1-yl)methyl-, (1,4-Dicyano-naphthalin-2-yl)methyl- oder (2,4-Dimethoxy-naphthalin-1-yl)methyl-Gruppe,
eine (Furan-2-yl)methyl-, (Furan-3-yl)methyl-, (5-Brom-furan-2-yl)methyl-, (5-Methyl-furan-2-yl)methyl-, (5-Cyano-furan-2-yl)methyl- oder (5-Methoxycarbonyl-furan-2-yl)methyl-Gruppe,
eine (Pyridin-2-yl)methyl-, (6-Fluor-pyridin-2-yl)methyl- oder (5-Methoxy-pyridin-2-yl)methyl-Gruppe,
eine (3-Cyanopyridin-2-yl)methyl-, (6-Cyanopyridin-2-yl)methyl-, (5-Cyano-pyridin-2-yl)methyl-, (4-Cyano-pyridin-2-yl)methyl-, (4-Cyano-pyridin-3-yl)methyl-, (3-Cyano-pyridin-4-yl)methyl-, (2-Cyano-pyridin-3-yl)methyl-, (2-Cyano-pyridin-4-yl)methyl-, (5-Cyano-pyridin-3-yl)methyl-, (6-Cyano-pyridin-3-yl)methyl- oder (5-Cyano-6-methoxy-pyridin-2-yl)methyl-Gruppe,
eine (6-Phenyl-pyridin-2-yl)methyl- oder eine ([2,2']Bipyridinyl-6-yl)methyl-Gruppe,
eine (Pyrimidin-2-yl)methyl-, (4-Methyl-pyrimidin-2-yl)methyl- oder (4,6-Dimethyl-pyrimidin-2-yl)methyl-Gruppe,
eine (2-Phenyl-pyrimidin-4-yl)methyl- oder (4-Phenyl-pyrimidin-2-yl)methyl-Gruppe,
eine [(1-Methyl-1H-benzotriazol-5-yl)methyl]-Gruppe,
eine (6-Fluor-chinolin-2-yl)methyl-, (7-Fluor-chinolin-2-yl)methyl-, (2-Methyl-chinolin-4-yl)methyl-, (3-Cyano-chinolin-2-yl)methyl-, (3-Cyano-4-methyl-chinolin-2-yl)methyl-, (4-Cyano-chinolin-2-yl)methyl-, (5-Cyano-chinolin-2-yl)methyl-, (8-Cyano-chinolin-2-yl)methyl-, (6-Amino-chinolin-2-yl)methyl-, (8-Amino-chinolin-2-yl)methyl-, (4-Methoxy-chinolin-2-yl)methyl-, (6-Methoxy-chinolin-2-yl)methyl-, (6,7-Dimethoxy-chinolin-2-yl)methyl- oder (8-Cyano-chinolin-7-yl)methyl-Gruppe,
eine (1-Cyano-isochinolin-3-yl)methyl-, (4-Cyano-isochinolin-1-yl)methyl- (4-Cyano-isochinolin-3-yl)methyl- oder [(4-(Pyridin-2-yl)-isochinolin-1-yl]methyl-Gruppe,
eine (Chinazolin-6-yl)methyl-, (Chinazolin-7-yl)methyl-, (2-Methyl-chinazolin-4-yl)methyl-, (4,5-Dimethyl-chinazolin-2-yl)methyl-, (4-Ethyl-chinazolin-2-yl)methyl-, (4-Cyclopropyl-chinazolin-2-yl)methyl-, (2-Phenyl-chinazolin-4-yl)methyl-, (4-Cyano-chinazolin-2-yl)methyl-, (4-Phenylamino-chinazolin-2-yl)methyl- oder (4-Benzylamino-chinazolin-2-yl)methyl-Gruppe,
eine (Chinoxalin-5-yl)methyl- (Chinoxalin-6-yl)methyl- oder (2,3-Dimethyl-chinoxalin-6-yl)methyl-Gruppe, oder
eine ([1,5]Naphthyridin-3-yl)methyl-Gruppe,
deren Tautomere, Enantiomere, Diastereomere, deren Gemische und deren Salze.

Bevorzugt sind Verbindungen der allgemeinen Formel 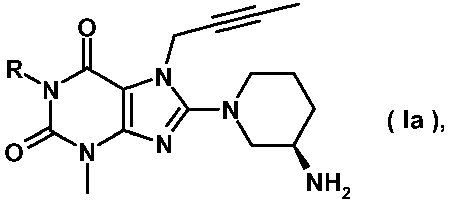 in der R wie oben erwähnt definiert ist, sowie deren Tautomere und Salze.

Ebenfalls bevorzugt sind Verbindungen der allgemeinen Formel 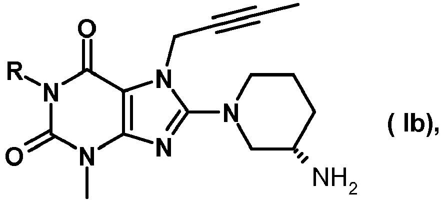 in der R wie oben erwähnt definiert ist, sowie deren Tautomere und Salze.

Beschreibungsgemäß erhält man die Verbindungen der allgemeinen Formel I nach an sich bekannten Verfahren, beispielsweise nach folgenden Verfahren:
a) Umsetzung einer Verbindung der allgemeinen Formel 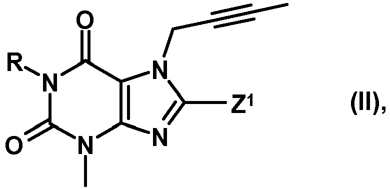 in der
   R wie eingangs erwähnt definiert ist und
   Z¹ eine Austrittsgruppe wie ein Halogenatom, eine substituierte Hydroxy-, Mercapto-, Sulfinyl-, Sulfonyl- oder Sulfonyloxygruppe wie ein Chlor- oder Bromatom, eine Methansulfonyl- oder Methansulfonyloxygruppe darstellt, mit 3-Aminopiperidin, dessen Enantiomeren oder dessen Salzen.

   Die Umsetzung wird zweckmäßigerweise in einem Lösungsmittel wie Isopropanol, Butanol, Tetrahydrofuran, Dioxan, Dimethylformamid, Dimethylsulfoxid, Ethylenglycolmonomethylether, Ethylenglycoldiethylether oder Sulfolan gegebenenfalls in Gegenwart einer anorganischen oder tertiären organischen Base, z.B. Natriumcarbonat, Kaliumcarbonat oder Kaliumhydroxid, einer tertiären organischen Base, z.B. Triethylamin, oder in Gegenwart von N-Ethyl-diisopropylamin (Hünig-Base), wobei diese organischen Basen gleichzeitig auch als Lösungsmittel dienen können, und gegebenenfalls in Gegenwart eines Reaktionsbeschleunigers wie einem Alkalihalogenid oder einem Katalysator auf Palladiumbasis bei Temperaturen zwischen -20 und 180°C, vorzugsweise jedoch bei Temperaturen zwischen -10 und 120°C, durchgeführt. Die Umsetzung kann jedoch auch ohne Lösungsmittel oder in einem Überschuß des 3-Aminopiperidins durchgeführt werden.
b) Entschützung einer Verbindung der allgemeinen Formel 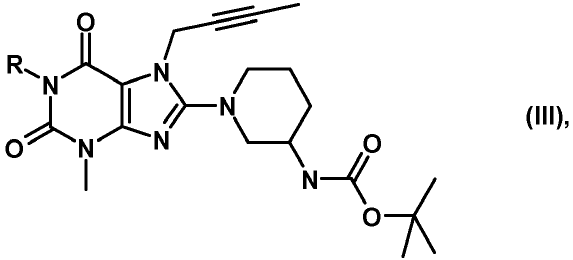 in der R wie eingangs definiert ist.

Die Abspaltung des tert.-Butyloxycarbonylrestes erfolgt vorzugsweise durch Behandlung mit einer Säure wie Trifluoressigsäure oder Salzsäure oder durch Behandlung mit Bromtrimethylsilan oder lodtrimethylsilan gegebenenfalls unter Verwendung eines Lösungsmittels wie Methylenchlorid, Essigester, Dioxan, Methanol, Isopropanol oder Diethylether bei Temperaturen zwischen 0 und 80°C.

Bei den vorstehend beschriebenen Umsetzungen können gegebenenfalls vorhandene reaktive Gruppen wie Amino-, Alkylamino- oder Iminogruppen während der Umsetzung durch übliche Schutzgruppen geschützt werden, welche nach der Umsetzung wieder abgespalten werden.

Beispielsweise kommen als Schutzreste für eine Amino-, Alkylamino- oder Iminogruppe die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran bei Temperaturen zwischen 0 und 50°C.

Die Abspaltung eines Phthalylrestes erfolgt vorzugsweise in Gegenwart von Hydrazin oder eines primären Amins wie Methylamin, Ethylamin, Ethanolamin oder n-Butylamin in einem Lösungsmittel wie Methanol, Ethanol, Isopropanol, Toluol/Wasser oder Dioxan bei Temperaturen zwischen 20 und 50°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-O-p-toluoyl-weinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Die als Ausgangsstoffe verwendeten Verbindungen der allgemeinen Formeln II und III sind entweder literaturbekannt oder man erhält diese nach an sich literaturbekannten Verfahren (siehe Beispiele I bis XXV).

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf das Enzym DPP-IV.

Die biologischen Eigenschaften der neuen Verbindungen wurden wie folgt geprüft:
Die Fähigkeit der Substanzen und ihrer entsprechenden Salze, die DPP-IV Aktivität zu hemmen, kann in einem Versuchsaufbau gezeigt werden, in dem ein Extrakt der humanen Koloncarcinomzelllinie Caco-2 als DPP IV Quelle benutzt wird. Die Differenzierung der Zellen, um die DPP-IV Expression zu induzieren, wurde nach der Beschreibung von Reiher et al. in einem Artikel mit dem Titel "Increased expression of intestinal cell line Caco-2" , erschienen in Proc. Natl. Acad. Sci. Vol. 90, Seiten 5757-5761 (1993), durchgeführt. Der Zellextrakt wurde von in einem Puffer (10 mM Tris HCl, 0.15 M NaCl, 0.04 t.i.u. Aprotinin, 0.5% Nonidet-P40, pH 8.0) solubilisierten Zellen durch Zentrifugation bei 35,000 g für 30 Minuten bei 4°C (zur Entfernung von Zelltrümmern) gewonnen.

Der DPP-IV Assay wurde wie folgt durchgeführt:
50 µl Substratlösung (AFC; AFC ist Amido-4-trifluormethylcoumarin), Endkonzentration 100 µM, wurden in schwarze Mikrotiterplatten vorgelegt. 20 µl Assay Puffer (Endkonzentrationen 50 mM Tris HCl pH 7.8, 50 mM NaCl, 1 % DMSO) wurde zupipettiert. Die Reaktion wurde durch Zugabe von 30 µl solubilisiertem Caco-2 Protein (Endkonzentration 0.14 µg Protein pro Well) gestartet. Die zu überprüfenden Testsubstanzen wurden typischerweise in 20 µl vorverdünnt zugefügt, wobei das Assaypuffervolumen dann entsprechend reduziert wurde. Die Reaktion wurde bei Raumtemperatur durchgeführt, die Inkubationsdauer betrug 60 Minuten. Danach wurde die Fluoreszenz in einem Victor 1420 Multilabel Counter gemessen, wobei die Anregungswellenlänge bei 405 nm und die Emissionswellenlänge bei 535 nm lag. Leerwerte (entsprechend 0 % Aktivität) wurden in Ansätzen ohne Caco-2 Protein (Volumen ersetzt durch Assay Puffer), Kontrollwerte (entsprechend 100 % Aktivität) wurden in Ansätzen ohne Substanzzusatz erhalten. Die Wirkstärke der jeweiligen Testsubstanzen, ausgedrückt als IC₅₀ Werte, wurden aus Dosis-Wirkungs Kurven berechnet, die aus jeweils 11 Meßpunkten bestanden. Hierbei wurden folgende Ergebnisse erhalten:

| Verbindung (Beispiel Nr.) | DPP IV-Hemmung IC₅₀ [nM] |
|---|---|
| 1 | 6 |
| 1(3) | 6 |
| 1(4) | 9 |
| 1(6) | 2 |
| 1(7) | 5 |
| 1(12) | 2 |
| 1(21) | 2 |
| 1(26) | 2 |
| 1(30) | 2 |
| 1(31) | 3 |
| 1(38) | 1 |
| 1(39) | 2 |

Die beschreibungsgemäß hergestellten Verbindungen sind gut verträglich, da beispielsweise nach oraler Gabe von 10 mg/kg der Verbindung des Beispiels 1(30) an Ratten keine Änderungen im Verhalten der Tiere beobachtet werden konnten.

Im Hinblick auf die Fähigkeit, die DPP-IV Aktivität zu hemmen, sind die erfindungsgemäßen Verbindungen der allgemeinen Formel I und ihre entsprechenden pharmazeutisch akzeptablen Salze geeignet, alle diejenigen Zustände oder Krankheiten zu beeinflussen, die durch eine Hemmung der DPP-IV Aktivität beeinflusst werden können. Es ist daher zu erwarten, daß die erfindungsgemäßen Verbindungen zur Prävention oder Behandlung von Krankheiten oder Zuständen wie Diabetes mellitus Typ 1 und Typ 2, Prädiabetes, Verminderung der Glukosetoleranz oder Veränderungen im Nüchternblutzucker, diabetische Komplikationen (wie z.B. Retinopathie, Nephropathie oder Neuropathien), metabolische Azidose oder Ketose, reaktiver Hypoglykämie, Insulinresistenz, Metabolischem Syndrom, Dyslipidämien unterschiedlichster Genese, Arthritis, Atherosklerose und verwandte Erkrankungen, Adipositas, Allograft Transplantation und durch Calcitonin verursachte Osteoporose geeignet sind. Darüberhinaus sind diese Substanzen geeignet, die B-Zelldegeneration wie z.B. Apoptose oder Nekrose von pankreatischen B-Zellen zu verhindern. Die Substanzen sind weiter geeignet, die Funktionalität von pankreatischen Zellen zu verbessern oder wiederherzustellen, daneben die Anzahl und Größe von pankreatischen B-Zellen zu erhöhen. Zusätzlich und begründet durch die Rolle der Glucagon-Like Peptide, wie z.B. GLP-1 und GLP-2 und deren Verknüpfung mit DPP-IV Inhibition, wird erwartet, daß die erfindungsgemäßen Verbindungen geeignet sind, um unter anderem einen sedierenden oder angstlösenden Effekt zu erzielen, darüberhinaus katabole Zustände nach Operationen oder hormonelle Stressantworten günstig zu beeinflussen oder die Mortalität und Morbidität nach Myokardinfarkt reduzieren zu können. Darüberhinaus sind sie geeignet zur Behandlung von allen Zuständen, die im Zusammenhang mit oben genannten Effekten stehen und durch GLP-1 oder GLP-2 vermittelt sind. Die erfindungsgemäßen Verbindungen sind ebenfalls als Diuretika oder Antihypertensiva einsetzbar und zur Prävention und Behandlung des akuten Nierenversagens geeignet. Weiterhin sind die erfindungsgemäßen Verbindungen zur Behandlung entzündlicher Erkrankungen der Atemwege einsetzbar. Ebenso sind sie zur Prävention und Therapie von chronischen entzündlichen Darmerkrankungen wie z.B. Reizdarmsyndrom (IBS), Morbus Crohn oder Colitis ulcerosa ebenso wie bei Pankreatitis geeignet. Des weiteren wird erwartet, daß sie bei jeglicher Art von Verletzung oder Beeinträchtigung im Gastrointestinaltrakt eingesetzt werden können wie auch z.B. bei Kolitiden und Enteriden. Darüberhinaus wird erwartet, daß DPP-IV Inhibitoren und somit auch die erfindungsgemäßen Verbindungen zur Behandlung der Unfruchtbarkeit oder zur Verbesserung der Fruchtbarkeit beim Menschen oder im Säugetierorganismus verwendet werden können, insbesondere dann, wenn die Unfruchtbarkeit im Zusammenhang mit einer Insulinresistenz oder mit dem polyzystischen Ovarialsyndrom steht. Auf der anderen Seite sind diese Substanzen geeignet, die Motilität der Spermien zu beeinflussen und sind damit als Kontrazeptiva zur Verwendung beim Mann einsetzbar. Des weiteren sind die Substanzen geeignet, Mangelzustände von Wachstumshormon, die mit Minderwuchs einhergehen, zu beeinflussen, sowie bei allen Indikationen sinnvoll eingesetzt werden können, bei denen Wachstumshormon verwendet werden kann. Die erfindungsgemäßen Verbindungen sind auf Grund ihrer Hemmwirkung gegen DPP IV auch geeignet zur Behandlung von verschiedenen Autoimmunerkrankungen wie z.B. rheumatoide Arthritis, Multiple Sklerose, Thyreoditiden und Basedow'scher Krankheit etc.. Darüberhinaus können sie eingesetzt werden bei viralen Erkrankungen wie auch z.B. bei HIV Infektionen, zur Stimulation der Blutbildung, bei benigner Prostatahyperplasie, bei Gingivitiden, sowie zur Behandlung von neuronalen Defekten und neurdegenerativen Erkrankungen wie z.B. Morbus Alzheimer. Beschriebene Verbindungen sind ebenso zu verwenden zur Therapie von Tumoren, insbesondere zur Veränderung der Tumorinvasion wie auch Metastatisierung, Beispiele hier sind die Anwendung bei T-Zell Lymphomen, akuter lymphoblastischer Leukämie, zellbasierende Schilddrüsenkarzinome, Basalzellkarzinome oder Brustkarzinome. Weitere Indikationen sind Schlaganfall, Ischämien verschiedenster Genese, Morbus Parkinson und Migräne. Darüberhinaus sind weitere Indikationsgebiete follikuläre und epidermale Hyperkeratosen, erhöhte Keratinozytenproliferation, Psoriasis, Enzephalomyelitiden, Glomerulonephritiden, Lipodystrophien, sowie psychosomatische, depressive und neuropsychiatrische Erkrankungen verschiedenster Genese.

Die erfindungsgemäßen Verbindungen können auch in Kombination mit anderen Wirkstoffen verwendet werden. Zu den zu einer solchen Kombination geeigneten Therapeutika gehören z.B. Antidiabetika, wie etwa Metformin, Sulfonylharnstoffe (z.B. Glibenclamid, Tolbutamid, Glimepiride), Nateglinide, Repaglinide, Thiazolidindione (z.B. Rosiglitazone, Pioglitazone), PPAR-gamma-Agonisten (z.B. Gl 262570) und -Antagonisten, PPAR-gamma/alpha Modulatoren (z.B. KRP 297), PPAR-gamma/alpha/delta Modulatoren, AMPK-Aktivatoren, ACC1 und ACC2 Inhibitoren, DGAT-Inhibitoren, SMT3-Rezeptor-Agonisten, 11β-HSD-Inhibitoren, FGF19-Agonisten oder -Mimetika, alpha-Glucosidasehemmer (z.B. Acarbose, Voglibose), andere DPPIV Inhibitoren, alpha2-Antagonisten, Insulin und Insulinanaloga, GLP-1 und GLP-1 Analoga (z.B. Exendin-4) oder Amylin. Daneben sind Kombinationen mit SGLT2-Inhibitoren wie T-1095 oder KGT-1251 (869682), Inhibitoren der Proteintyrosinphosphatase 1, Substanzen, die eine deregulierte Glucoseproduktion in der Leber beeinflussen, wie z.B. Inhibitoren der Glucose-6-phosphatase, oder der Fructose-1,6-bisphosphatase, der Glycogenphosphorylase, Glucagonrezeptor Antagonisten und Inhibitoren der Phosphoenolpyruvatcarboxykinase, der Glykogensynthasekinase oder der Pyruvatdehydrokinase, Lipidsenker, wie etwa HMG-CoA-Reduktasehemmer (z.B. Simvastatin, Atorvastatin), Fibrate (z.B. Bezafibrat, Fenofibrat), Nikotinsäure und deren Derivate, PPAR-alpha Agonisten, PPAR-delta Agonisten, ACAT Inhibitoren (z.B. Avasimibe) oder Cholesterolresorptionsinhibitoren wie zum Beispiel Ezetimibe, gallensäurebindende Substanzen wie zum Beispiel Colestyramin, Hemmstoffe des ilealen Gallensäuretransportes, HDL-erhöhende Verbindungen wie zum Beispiel Inhibitoren von CETP oder Regulatoren von ABC1 oder LXRalpha Antagonisten, LXRbeta Agonisten oder LXRalpha/beta Regulatoren oder Wirkstoffe zur Behandlung von Obesitas, wie etwa Sibutramin oder Tetrahydrolipstatin, Dexfenfluramin, Axokine, Antagonisten des Cannbinoid1 Rezeptors, MCH-1 Rezeptorantagonisten, MC4 Rezeptor Agonisten, NPY5 oder NPY2 Antagonisten oder β₃-Agonisten wie SB-418790 oder AD-9677 ebenso wie Agonisten des 5HT2c Rezeptors möglich.

Daneben ist eine Kombination mit Medikamenten zur Beeinflussung des Bluthochdrucks wie z.B. All Antagonisten oder ACE Inhibitoren, Diuretika, β-Blocker, Ca-Antagonisten und anderen oder Kombinationen daraus geeignet.

Die zur Erzielung einer entsprechenden Wirkung erforderliche Dosierung beträgt zweckmäßigerweise bei intravenöser Gabe 1 bis 100 mg, vorzugsweise 1 bis 30 mg, und bei oraler Gabe 1 bis 1000 mg, vorzugsweise 1 bis 100 mg, jeweils 1 bis 4 x täglich. Hierzu lassen sich die beschreibungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen einarbeiten.

Die nachfolgenden Beispiele sollen die Erfindung näher erläutern:
Herstellung der Ausgangsverbindungen

### Beispiel I

### 1-[(4-Phenylamino-chinazolin-2-yl)methyl]-3-methyl-7-(2-butin-1-yl)-8-[3-(tert.-butyloxycarbonylamino)-piperidin-1-yl]-xanthin

Ein Gemisch aus 416 mg 3-Methyl-7-(2-butin-1-yl)-8-[3-(tert.-butyloxycarbonylamino)-piperidin-1-yl]-xanthin und 456 mg Cäsiumcarbonat in 4 ml N,N-Dimethylformamid wird bei 80°C für 10 Minuten gerührt, dann werden 324 mg 2-Chlormethyl-4-phenyl-amino-chinazolin zugegeben und das Reaktionsgemisch wird zwei Stunden bei 80°C gerührt. Dann werden erneut 50 mg Cäsiumcarbonat und 50 mg Chlormethyl-4-phenylamino-chinazolin zugegeben und das Gemisch wird weitere 1.5 Stunden bei 80°C gerührt. Anschließend wird das Lösungsmittel abdestilliert und der Rückstand zwischen Wasser und Essigester verteilt. Die organische Phase wird mit verdünnter Zitronensäure, Wasser und gesättigter Natriumchloridlösung gewaschen, über Magnesiumsulfat getrocknet und eingeengt. Das Rohprodukt wird chromatographisch über eine Kieselgelsäule mit Essigester/Petrolether (8:2 auf 10:0) als Laufmittel gereinigt.
Ausbeute: 425 mg (65 % der Theorie)
R_{f}-Wert: 0.33 (Kieselgel, Essigester)
Massenspektrum (ESI⁺): m/z = 650 [M+H]⁺

Analog Beispiel I werden folgende Verbindungen erhalten:
(1) 1-[(4-Benzylamino-chinazolin-2-yl)methyl]-3-methyl-7-(2-butin-1-yl)-8-[3-(tert.-butyloxycarbonylamino)-piperidin-1-yl]-xanthin
   R_{f}-Wert: 0.20 (Kieselgel, Essigester)
   Massenspektrum (ESI⁺): m/z = 664 [M+H]⁺
(2) 1-[(2-Methyl-chinolin-4-yl)methyl]-3-methyl-7-(2-butin-1-yl)-8-[(*R*)-3-(tert.-butyloxycarbonylamino)-piperidin-1-yl]-xanthin
   R_{f}-Wert: 0.80 (Kieselgel, Methylenchlorid/Methanol = 9:1)
   Massenspektrum (ESI⁺): m/z = 572 [M+H]⁺
(3) 1-[(3-Cyano-naphthalin-1-yl)methyl]-3-methyl-7-(2-butin-1-yl)-8-[(R)-3-(tert.-butyloxycarbonylamino)-piperidin-1-yl]-xanthin
   R_{f}-Wert: 0.67 (Kieselgel, Methylenchlorid/Methanol = 95:5)
   Massenspektrum (ESI⁺): m/z = 582 [M+H]⁺
(4) 1-[(2-Phenyl-chinazolin-4-yl)methyl]-3-methyl-7-(2-butin-1-yl)-8-[(*R*)-3-(tert.-butyloxycarbonylamino)-piperidin-1-yl]-xanthin
   R_{f}-Wert: 0.75 (Kieselgel, Methylenchlorid/Essigester = 1:1)
   Massenspektrum (ESI⁺): m/z = 635 [M+H]⁺
(5) 1-[(4-Cyano-isochinolin-1-yl)methyl]-3-methyl-7-(2-butin-1-yl)-8-[(*R*)-3-(tert.-butyloxycarbonylamino)-piperidin-1-yl]-xanthin
   R_{f}-Wert: 0.75 (Kieselgel, Methylenchlorid/Methanol/konz. wässriges Ammoniak = 90:10:1)
   Massenspektrum (ESI⁺): m/z = 583 [M+H]⁺
(6) 1-[(4-Cyano-chinolin-2-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 583 [M+H]⁺
(7) 1-[2-(3-Cyclopropyloxy-phenyl)-2-oxo-ethyl]-3-methyl-7-(2-butin-1-yl)-8-[(*R*)-3-(tert.-butyloxycarbonylamino)-piperidin-1-yl]-xanthin
   R_{f}-Wert: 0.75 (Kieselgel, Methylenchlorid/Essigester = 1:1)
   Massenspektrum (ESI⁺): m/z = 591 [M+H]⁺
(8) 1-[2-(3-Cyclopropylmethoxy-phenyl)-2-oxo-ethyl]-3-methyl-7-(2-butin-1-yl)-8-[(*R*)-3-(tert.-butyloxycarbonylamino)-piperidin-1-yl]-xanthin
   R_{f}-Wert: 0.65 (Kieselgel, Methylenchlorid/Essigester = 1:1)
   Massenspektrum (ESI⁺): m/z = 605 [M+H]⁺
(9) 1-[2-(3-Cyclobutyloxy-phenyl)-2-oxo-ethyl]-3-methyl-7-(2-butin-1-yl)-8-[(*R*)-3-(tert.-butyloxycarbonylamino)-piperidin-1-yl]-xanthin
   R_{f}-Wert: 0.85 (Kieselgel, Methylenchlorid/Essigester = 1:1)
   Massenspektrum (ESI⁺): m/z = 605 [M+H]⁺
(10) 1-[(1-Cyano-isochinolin-3-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 583 [M+H]⁺
(11) 1-[(2,4-Methoxy-naphthalin-1-yl)methyl]-3-methyl-7-(2-butin-1-yl)-8-[(*R*)-3-(tert.-butyloxycarbonylamino)-piperidin-1-yl]-xanthin
   R_{f}-Wert: 0.70 (Kieselgel, Essigester)
   Massenspektrum (ESI⁺): m/z = 617 [M+H]⁺
(12) 1-[(2,3-Dimethyl-chinoxalin-6-yl)methyl]-3-methyl-7-(2-butin-1-yl)-8-[(*R*)-3-(tert.-butyloxycarbonylamino)-piperidin-1-yl]-xanthin
   R_{f}-Wert: 0.50 (Kieselgel, Essigester)
   Massenspektrum (ESI⁺): m/z = 587 [M+H]⁺
(13) 1-[(6-Nitro-chinolin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.45 (Kieselgel, Essigester/Petrolether = 7:3)
   Massenspektrum (ESI⁺): m/z = 603 [M+H]⁺
(14) 1-[(Chinoxalin-5-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 559 [M+H]⁺
(15) 1-[(6-Methoxy-chinolin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.65 (Kieselgel, Essigester)
   Massenspektrum (ESI⁺): m/z = 588 [M+H]⁺
(16) 1-[(6-Phenyl-pyridin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.43 (Kieselgel, Methylenchlorid/Methanol = 96:4)
   Massenspektrum (ESI⁺): m/z = 584 [M+H]⁺
(17) 1-{[(4-(Pyridin-2-yl)-isochinolin-1-yl]methyl}-3-methyl-7-(2-butin-1-yl)-8-[(*R*)-3-(tert.-butyloxycarbonylamino)-piperidin-1-yl]-xanthin
(18) 1-[(7-Fluor-chinolin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.24 (Kieselgel, Essigester/Petrolether = 1:1)
   Massenspektrum (ESI⁺): m/z = 576 [M+H]⁺
(19) 1-[(8-Nitro-chinolin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.63 (Kieselgel, Methylenchlorid/Methanol = 95:5)
   Massenspektrum (ESI⁺): m/z = 603 [M+H]⁺
(20) 1-[(6-Fluor-chinolin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.47 (Kieselgel, Methylenchlorid/Methanol = 95:5)
   Massenspektrum (ESI⁺): m/z = 576 [M+H]⁺
(21) 1-[2-Oxo-2-(2-brom-phenyl)-ethyl]-3-methyl-7-(2-butin-1-yl)-8-[(*R*)-3-(tert.-butyloxycarbonylamino)-piperidin-1-yl]-xanthin
   R_{f}-Wert: 0.75 (Kieselgel, Methylenchlorid/Essigester = 1:1)
   Massenspektrum (ESI⁺): m/z = 613, 615 [M+H]⁺
(22) 1-Cyanomethyl-3-methyl-7-(2-butin-1-yl)-8-[(*R*)-3-(tert.-butyloxycarbonylamino)-piperidin-1-yl]-xanthin
   R_{f}-Wert: 0.80 (Kieselgel, Essigester)
   Massenspektrum (ESI⁺): m/z = 456 [M+H]⁺
(23) 1-[(4-Methoxy-chinolin-2-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 588 [M+H]⁺
(24) 1-[(2-Phenyl-pyrimidin-4-yl)methyl]-3-methyl-7-(2-butin-1-yl)-8-[(*R*)-3-(tert.-butyloxycarbonylamino)-piperidin-1-yl]-xanthin
   R_{f}-Wert: 0.39 (Kieselgel, Methylenchlorid/Methanol = 96:4)
   Massenspektrum (ESI⁺): m/z = 585 [M+H]⁺
(25) 1-[([1,5]Naphthyridin-3-yl)methyl]-3-methyl-7-(2-butin-1-yl)-8-[(*R*)-3-(tert.-butyloxycarbonylamino)-piperidin-1-yl]-xanthin
   R_{f}-Wert: 0.28 (Kieselgel, Essigester)
   Massenspektrum (ESI⁺): m/z = 559 [M+H]⁺
(26) 1-[(3-Cyano-4-methyl-chinolin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.75 (Kieselgel, Methylenchlorid/Essigester = 1:1)
   Massenspektrum (ESI⁺): m/z = 597 [M+H]⁺
(27) 1-[(4,5-Dimethyl-chinazolin-2-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 587 [M+H]⁺
(28) 1-[(5-Cyano-chinolin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.42 (Kieselgel, Petrolether/Essigester = 1:2)
   Massenspektrum (ESI⁺): m/z = 583 [M+H]⁺
(29) 1-[(3-Cyano-chinolin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.80 (Kieselgel, Methylenchlorid/Essigester = 1:1)
(30) 1-[(4-Phenyl-pyrimidin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.46 (Kieselgel, Essigester)
(31) 1-[2-(2-Nitro-phenyl)-2-oxo-ethyl]-3-methyl-7-(2-butin-1-yl)-8-[(*R*)-3-(tert.-butyloxycarbonylamino)-piperidin-1-yl]-xanthin
   R_{f}-Wert: 0.75 (Kieselgel, Essigester)
   Massenspektrum (ESI⁺): m/z = 580 [M+H]⁺
(32) 1-[(1,4-Dicyano-naphthalin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.54 (Kieselgel, Methylenchlorid/Methanol = 95:5)
   Massenspektrum (ESI⁺): m/z = 607 [M+H]⁺
(33) 1-[(6,7-Dimethoxy-chinolin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.36 (Kieselgel, Essigester)
   Massenspektrum (ESI⁺): m/z = 618 [M+H]⁺
(34) 1-[(Chinazolin-6-yl)methyl]-3-methyl-7-(2-butin-1-yl)-8-[(*R*)-3-(tert.-butyloxycarbonylamino)-piperidin-1-yl]-xanthin
   R_{f}-Wert: 0.20 (Kieselgel, Essigester)
   Massenspektrum (ESI⁺): m/z = 559 [M+H]⁺
(35) 1-[(4-Cyano-chinazolin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.40 (Kieselgel, Methylenchlorid/Essigester = 7:3)
   Massenspektrum (ESI⁺): m/z = 584 [M+H]⁺
(36) 1-[(Chinazolin-7-yl)methyl]-3-methyl-7-(2-butin-1-yl)-8-[(*R*)-3-(tert.-butyloxycarbonylamino)-piperidin-1-yl]-xanthin
   R_{f}-Wert: 0.20 (Kieselgel, Essigester)
   Massenspektrum (ESI⁺): m/z = 559 [M+H]⁺
(37) 1-(2-Cyano-benzyl)-3-methyl-7-(2-butin-1-yl)-8-[(*R*)-3-(tert.-butyloxycarbonylamino)-piperidin-1-yl]-xanthin
   R_{f}-Wert: 0.50 (Kieselgel, Methylenchlorid/Essigester = 7:3)
   Massenspektrum (ESI⁺): m/z = 532 [M+H]⁺
(38) 1-(3-Cyano-benzyl)-3-methyl-7-(2-butin-1-yl)-8-[(*R*)-3-(tert.-butyloxycarbonylamino)-piperidin-1-yl]-xanthin
   R_{f}-Wert: 0.58 (Kieselgel, Methylenchlorid/Essigester = 1:1)
   Massenspektrum (ESI⁺): m/z = 532 [M+H]⁺
(39) 1-(4-Cyano-benzyl)-3-methyl-7-(2-butin-1-yl)-8-[(*R*)-3-(tert.-butyloxycarbonylamino)-piperidin-1-yl]-xanthin
   R_{f}-Wert: 0.61 (Kieselgel, Methylenchlorid/Essigester = 1:1)
   Massenspektrum (ESI⁺): m/z = 532 [M+H]⁺
(40) 1-[(Pyridin-2-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 508 [M+H]⁺
(41) 1-Benzyl-3-methyl-7-(2-butin-1-yl)-8-[(*R*)-3-(tert.-butyloxycarbonylamino)-piperidin-1-yl]-xanthin
   R_{f}-Wert: 0.70 (Kieselgel, Methylenchlorid/Essigester = 1:1)
   Massenspektrum (ESI⁺): m/z = 507 [M+H]⁺
(42) 1-(4-Methoxy-benzyl)-3-methyl-7-(2-butin-1-yl)-8-[(*R*)-3-(tert.-butyloxycarbonylamino)-piperidin-1-yl]-xanthin
   R_{f}-Wert: 0.75 (Kieselgel, Essigester)
   Massenspektrum (ESI⁺): m/z = 537 [M+H]⁺
(43) 1-(2-Chlor-benzyl)-3-methyl-7-(2-butin-1-yl)-8-[(*R*)-3-(tert.-butyloxycarbonylamino)-piperidin-1-yl]-xanthin
   R_{f}-Wert: 0.80 (Kieselgel, Essigester)
   Massenspektrum (ESI⁺): m/z = 541, 543 [M+H]⁺
(44) 1-(2,6-Dicyano-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 557 [M+H]⁺
(45) 1-(2-Cyano-4-brom-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 610, 612 [M+H]⁺
(46) 1-(3-Fluor-benzyl)-3-methyl-7-(2-butin-1-yl)-8-[(*R*)-3-(tert.-butyloxycarbonylamino)-piperidin-1-yl]-xanthin
   R_{f}-Wert: 0.80 (Kieselgel, Essigester)
   Massenspektrum (ESI⁺): m/z = 525 [M+H]⁺
(47) 1-(3,5-Dimethoxy-benzyl)-3-methyl-7-(2-butin-1-yl)-8-[(*R*)-3-(tert.-butyloxycarbonylamino)-piperidin-1-yl]-xanthin
   R_{f}-Wert: 0.70 (Kieselgel, Essigester)
   Massenspektrum (ESI⁺): m/z = 567 [M+H]⁺
(48) 1-(2-Fluor-benzyl)-3-methyl-7-(2-butin-1-yl)-8-[(*R*)-3-(tert.-butyloxycarbonylamino)-piperidin-1-yl]-xanthin
   R_{f}-Wert: 0.85 (Kieselgel, Essigester)
   Massenspektrum (ESI⁺): m/z = 525 [M+H]⁺
(49) 1-[(6-Cyano-pyridin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.60 (Kieselgel, Methylenchlorid/Essigester = 1:1)
   Massenspektrum (ESI⁺): m/z = 533 [M+H]⁺
(50) 1-[(3-Cyano-pyridin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.60 (Kieselgel, Methylenchlorid/Essigester = 1:1)
   Massenspektrum (ESI⁺): m/z = 533 [M+H]⁺
(51) 1-(2-Cyano-3-chlor-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 566, 568 [M+H]⁺
(52) 1-(4-Fluor-benzyl)-3-methyl-7-(2-butin-1-yl)-8-[(*R*)-3-(tert.-butyloxycarbonylamino)-piperidin-1-yl]-xanthin
   R_{f}-Wert: 0.80 (Kieselgel, Essigester)
   Massenspektrum (ESI⁺): m/z = 525 [M+H]⁺
(53) 1-(4-Chlor-benzyl)-3-methyl-7-(2-butin-1-yl)-8-[(*R*)-3-(tert.-butyloxycarbonylamino)-piperidin-1-yl]-xanthin
   R_{f}-Wert: 0.80 (Kieselgel, Essigester)
   Massenspektrum (ESI⁺): m/z = 541, 543 [M+H]⁺
(54) 1-(2-Cyano-4-fluor-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 550 [M+H]⁺
(55) 1-(3-Cyano-4-fluor-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 550 [M+H]⁺
(56) 1-(2-Chlor-4-cyano-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 566, 568 [M+H]⁺
(57) 1-[(5-Methoxycarbonyl-furan-2-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 555 [M+H]⁺
(58) 1-(2-Trifluormethyl-4-cyano-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 600 [M+H]⁺
(59) 1-(3,5-Dicyano-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 557 [M+H]⁺
(60) 1-(3-Nitro-4-cyano-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 577 [M+H]⁺
(61) 1-[(2-Cyano-pyridin-3-yl)methyl]-3-methyl-7-(2-butin-1-yl)-8-[(*R*)-3-(tert.-butyloxycarbonylamino)-piperidin-1-yl]-xanthin
   R_{f}-Wert: 0.50 (Kieselgel, Methylenchlorid/Essigester = 1:1)
   Massenspektrum (ESI⁺): m/z = 533 [M+H]⁺
(62) 1-(2-Cyano-4-methoxy-benzyl)-3-methyl-7-(2-butin-1-yl)-8-[(*R*)-3-(tert.-butyloxycarbonylamino)-piperidin-1-yl]-xanthin
   R_{f}-Wert: 0.70 (Kieselgel, Methylenchlorid/Essigester = 1:1)
   Massenspektrum (ESI⁺): m/z = 562 [M+H]⁺
(63) 1-(2-Cyano-5-methoxy-benzyl)-3-methyl-7-(2-butin-1-yl)-8-[(*R*)-3-(tert.-butyloxycarbonylamino)-piperidin-1-yl]-xanthin
   R_{f}-Wert: 0.75 (Kieselgel, Methylenchlorid/Essigester = 1:1)
   Massenspektrum (ESI⁺): m/z = 562 [M+H]⁺
(64) 1-(3-Methoxy-benzyl)-3-methyl-7-(2-butin-1-yl)-8-[(*R*)-3-(tert.-butyloxycarbonylamino)-piperidin-1-yl]-xanthin
   R_{f}-Wert: 0.80 (Kieselgel, Essigester)
   Massenspektrum (ESI⁺): m/z = 537 [M+H]⁺
(65) 1-(3-Trifluormethyl-benzyl)-3-methyl-7-(2-butin-1-yl)-8-[(*R*)-3-(tert.-butyloxycarbonylamino)-piperidin-1-yl]-xanthin
   R_{f}-Wert: 0.80 (Kieselgel, Essigester)
   Massenspektrum (ESI⁺): m/z = 575 [M+H]⁺
(66) 1-(3,4-Dimethoxy-benzyl)-3-methyl-7-(2-butin-1-yl)-8-[(*R*)-3-(tert.-butyloxycarbonylamino)-piperidin-1-yl]-xanthin
   R_{f}-Wert: 0.65 (Kieselgel, Essigester)
   Massenspektrum (ESI⁺): m/z = 567 [M+H]⁺
(67) 1-(3-Chlor-benzyl)-3-methyl-7-(2-butin-1-yl)-8-[(*R*)-3-(tert.-butyloxycarbonylamino)-piperidin-1-yl]-xanthin
   R_{f}-Wert: 0.80 (Kieselgel, Essigester)
   Massenspektrum (ESI⁺): m/z = 541, 543 [M+H]⁺
(68) 1-(4-Trifluormethyl-benzyl)-3-methyl-7-(2-butin-1-yl)-8-[(*R*)-3-(tert.-butyloxycarbonylamino)-piperidin-1-yl]-xanthin
   R_{f}-Wert: 0.85 (Kieselgel, Essigester)
   Massenspektrum (ESI⁺): m/z = 575 [M+H]⁺
(69) 1-[([2,2']Bipyridinyl-6-yl)methyl]-3-methyl-7-(2-butin-1-yl)-8-[(*R*)-3-(tert.-butyloxycarbonylamino)-piperidin-1-yl]-xanthin
   R_{f}-Wert: 0.53 (Aluminiumoxid, Methylenchlorid/Methanol = 98:2)
   Massenspektrum (ESI⁺): m/z = 585 [M+H]⁺
(70) 1-(3,4-Dimethoxy-6-fluor-benzyl)-3-methyl-7-(2-butin-1-yl)-8-[(*R*)-3-(tert.-butyloxycarbonylamino)-piperidin-1-yl]-xanthin
   R_{f}-Wert: 0.65 (Kieselgel, Essigester)
   Massenspektrum (ESI⁺): m/z = 585 [M+H]⁺
(71) 1-[(6-Fluor-pyridin-2-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 526 [M+H]⁺
(72) 1-[(5-Cyano-6-methoxy-pyridin-2-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 563 [M+H]⁺
(73) 1-(2,6-Difluor-benzyl)-3-methyl-7-(2-butin-1-yl)-8-[(R)-3-(tert.-butyloxycarbonylamino)-piperidin-1-yl]-xanthin
   R_{f}-Wert: 0.62 (Kieselgel, Essigester)
   Massenspektrum (ESI⁺): m/z = 543 [M+H]⁺
(74) 1-(3-Trifluormethoxy-benzyl)-3-methyl-7-(2-butin-1-yl)-8-[(*R*)-3-(tert.-butyloxycarbonylamino)-piperidin-1-yl]-xanthin
   R_{f}-Wert: 0.67 (Kieselgel, Essigester)
   Massenspektrum (ESI⁺): m/z = 591 [M+H]⁺
(75) 1-(4-Trifluormethoxy-benzyl)-3-methyl-7-(2-butin-1-yl)-8-[(*R*)-3-(tert.-butyloxycarbonylamino)-piperidin-1-yl]-xanthin
   R_{f}-Wert: 0.62 (Kieselgel, Essigester)
   Massenspektrum (ESI⁺): m/z = 591 [M+H]⁺
(76) 1-[(2-Cyano-pyridin-4-yl)methyl]-3-methyl-7-(2-butin-1-yl)-8-[(*R*)-3-(tert.-butyloxycarbonylamino)-piperidin-1-yl]-xanthin
   R_{f}-Wert: 0.55 (Kieselgel, Methylenchlorid/Essigester = 1:1)
   Massenspektrum (ESI⁺): m/z = 533 [M+H]⁺
(77) 1-[(5-Cyano-pyridin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.55 (Kieselgel, Methylenchlorid/Essigester = 1:1)
   Massenspektrum (ESI⁺): m/z = 533 [M+H]⁺
(78) 1-[(Pyrimidin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.60 (Kieselgel, Essigester/Methanol = 9:1)
   Massenspektrum (ESI⁺): m/z = 509 [M+H]⁺
(79) 1-[(4-Methyl-pyrimidin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.60 (Kieselgel, Essigester/Methanol = 9:1)
   Massenspektrum (ESI⁺): m/z = 523 [M+H]⁺
(80) 1-[(4,6-Dimethyl-pyrimidin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.70 (Kieselgel, Essigester/Methanol = 9:1)
   Massenspektrum (ESI⁺): m/z = 537 [M+H]⁺
(81) 1-[(Chinoxalin-6-yl)methyl]-3-methyl-7-(2-butin-1-yl)-8-brom-xanthin
   R_{f}-Wert: 0.55 (Kieselgel, Essigester)
   Massenspektrum (ESI⁺): m/z = 439, 441 [M+H]⁺
(82) 1-(3-Fluor-4-methoxy-benzyl)-3-methyl-7-(2-butin-1-yl)-8-[(*R*)-3-(tert.-butyloxycarbonylamino)-piperidin-1-yl]-xanthin
   R_{f}-Wert: 0.70 (Kieselgel, Essigester)
   Massenspektrum (ESI⁺): m/z = 555 [M+H]⁺
(83) 1-(3,4-Difluor-benzyl)-3-methyl-7-(2-butin-1-yl)-8-[(R)-3-(tert.-butyloxycarbonylamino)-piperidin-1-yl]-xanthin
   R_{f}-Wert: 0.75 (Kieselgel, Essigester)
   Massenspektrum (ESI⁺): m/z = 543 [M+H]⁺
(84) 1-(2-Fluor-5-methoxy-benzyl)-3-methyl-7-(2-butin-1-yl)-8-[(*R*)-3-(tert.-butyloxycarbonylamino)-piperidin-1-yl]-xanthin
   R_{f}-Wert: 0.55 (Kieselgel, Essigester/Petrolether = 3:2)
   Massenspektrum (ESI⁺): m/z = 555 [M+H]⁺
(85) 1-(2-Fluor-3-methoxy-benzyl)-3-methyl-7-(2-butin-1-yl)-8-[(*R*)-3-(tert.-butyloxycarbonylamino)-piperidin-1-yl]-xanthin
   R_{f}-Wert: 0.48 (Kieselgel, Essigester/Petrolether = 3:2)
   Massenspektrum (ESI⁺): m/z = 555 [M+H]⁺
(86) 1-[(4-Cyano-isochinolin-3-yl)methyl]-3-methyl-7-(2-butin-1-yl)-8-[(*R*)-3-(tert.-butyloxycarbonylamino)-piperidin-1-yl]-xanthin
   R_{f}-Wert: 0.55 (Kieselgel, Methylenchlorid/Methanol= 95:5)
   Massenspektrum (ESI⁺): m/z = 583 [M+H]⁺
(87) 1-(2-Fluor-4-methoxy-benzyl)-3-methyl-7-(2-butin-1-yl)-8-[(*R*)-3-(tert.-butyloxycarbonylamino)-piperidin-1-yl]-xanthin
   R_{f}-Wert: 0.48 (Kieselgel, Essigester/Petrolether = 1:1)
   Massenspektrum (ESI⁺): m/z = 555 [M+H]⁺
(88) 1-[(Furan-2-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 497 [M+H]⁺
(89) 1-(3,4-Dicyano-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 557 [M+H]⁺
(90) 1-(4-Cyano-2-fluor-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 550 [M+H]⁺
(91) (1-(2-Cyano-5-fluor-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 550 [M+H]⁺
(92) 1-[(5-Formyl-furan-2-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 525 [M+H]⁺
(93) 1-(2-Cyano-6-fluor-benzyl)-3-methyl-7-(2-butin-1-yl)-8-[(*R*)-3-(tert.-butyloxycarbonylamino)-piperidin-1-yl]-xanthin
(94) 1-(4-Cyano-3-fluor-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 550 [M+H]⁺
(95) 1-(2-Cyano-3-methoxy-benzyl)-3-methyl-7-(2-butin-1-yl)-8-brom-xanthin
   R_{f}-Wert: 0.85 (Kieselgel, Essigester)
   Massenspektrum (ESI⁺): m/z = 442, 444 [M+H]⁺
(96) 1-[(8-Cyano-chinolin-7-yl)methyl]-3-methyl-7-(2-butin-1-yl)-8-[(*R*)-3-(tert.-butyloxycarbonylamino)-piperidin-1-yl]-xanthin
   R_{f}-Wert: 0.70 (Kieselgel, Essigester)
   Massenspektrum (ESI⁺): m/z = 583 [M+H]⁺
(97) 1-[(4-Cyano-pyridin-3-yl)methyl]-3-methyl-7-(2-butin-1-yl)-8-[(*R*)-3-(tert.-butyloxycarbonylamino)-piperidin-1-yl]-xanthin
   R_{f}-Wert: 0.40 (Kieselgel, Essigester/Cyclohexan = 3:1)
   Massenspektrum (ESI⁺): m/z = 533 [M+H]⁺
(98) 1-[(8-Cyano-chinolin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.40 (Kieselgel, Essigester/Petrolether = 4:1)
   Massenspektrum (ESI⁺): m/z = 583 [M+H]⁺
(99) 1-[(1-Methyl-1H-benzotriazol-5-yl)methyl]-3-methyl-7-(2-butin-1-yl)-8-[(*R*)-3-(tert.-butyloxycarbonylamino)-piperidin-1-yl]-xanthin
   R_{f}-Wert: 0.30 (Kieselgel, Methylenchlorid/Essigester = 1:1)
   Massenspektrum (ESI⁺): m/z = 562 [M+H]⁺
(100) 1-[(3-Cyano-pyridin-4-yl)methyl]-3-methyl-7-(2-butin-1-yl)-8-[(*R*)-3-(tert.-butyloxycarbonylamino)-piperidin-1-yl]-xanthin
   R_{f}-Wert: 0.30 (Kieselgel, Methylenchlorid/Essigester = 1:1)
   Massenspektrum (ESI⁺): m/z = 533 [M+H]⁺
(101) 1-[(3-Cyano-pyridin-2-yl)methyl]-3-methyl-7-(2-butin-1-yl)-8-brom-xanthin Massenspektrum (ESI⁺): m/z = 413, 415 [M+H]⁺
(102) 1-[(4-Cyano-benzo[1,3]dioxol-5-yl)methyl]-3-methyl-7-(2-butin-1-yl)-8-[(*R*)-3-(tert.-butyloxycarbonylamino)-piperidin-1-yl]-xanthin
   R_{f}-Wert: 0.80 (Kieselgel, Essigester)
   Massenspektrum (ESI⁺): m/z = 576 [M+H]⁺

### Beispiel II

### 3-Methyl-7-(2-butin-1-yl)-8-[(R)-3-(tert.-butyloxycarbonylamino)-piperidin-1-yl]-xanthin

Zu 15.00 g 3-Methyl-7-(2-butin-1-yl)-8-brom-xanthin und 16.00 g Kaliumcarbonat in 100 ml Dimethylsulfoxid werden 11.00 g (*R*)-3-tert.-Butyloxycarbonylamino-piperidin gegeben und die dicke hellbeige Suspension wird vier Stunden mit einem mechanischen Rührer bei ca. 114°C gerührt. Dann werden nochmals 900 mg (R)-3-tert.-Butyloxycarbonylamino-piperidin, gelöst in 10 ml Dimethylsulfoxid, zum Reaktionsgemisch gegeben und dieses wird weitere zwei Stunden bei 114°C gerührt. Nach Abkühlung auf Raumtemperatur wird das Reaktionsgemisch mit reichlich Wasser verdünnt. Der entstandene Niederschlag wird gründlich verrieben, bis keine Klumpen mehr vorhanden sind, und absaugt. Der helle Feststoff wird erneut mit Wasser aufgeschlämmt, abgesaugt, mit Wasser und Diethylether nachgewaschen und im Umlufttrockenschrank bei 60°C getrocknet.
Ausbeute: 19.73 g (94 % der Theorie)
R_{f}-Wert: 0.64 (Kieselgel, Essigester)
Massenspektrum (ESI⁺): m/z = 417 [M+H]⁺

Analog Beispiel II wird folgende Verbindung erhalten:
(1) 3-Methyl-7-(2-butin-1-yl)-8-[(3-(tert.-butyloxycarbonylamino)-piperidin-1-yl]-xanthin
   Schmelzpunkt: 235-237°C
   Massenspektrum (ESI⁺): m/z = 417 [M+H]⁺
(2) 1-[(Chinoxalin-6-yl)methyl]-3-methyl-7-(2-butin-1-yl)-8-[(*R*)-3-(tert.-butyloxycarbonylamino)-piperidin-1-yl]-xanthin
   R_{f}-Wert: 0.40 (Kieselgel, Essigester)
   Massenspektrum (ESI⁺): m/z = 559 [M+H]⁺
(3) 1-[(5-Methyl-furan-2-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 511 [M+H]⁺
(4) 1-(2-Cyano-3-methoxy-benzyl)-3-methyl-7-(2-butin-1-yl)-8-[(*R*)-3-(tert.-butyloxycarbonylamino)-piperidin-1-yl]-xanthin
   R_{f}-Wert: 0.50 (Kieselgel, Essigester)
   Massenspektrum (ESI⁺): m/z = 562 [M+H]⁺
(5) 1-[(3-Cyano-pyridin-2-yl)methyl]-3-methyl-7-(2-butin-1-yl)-8-[(*S*)-3-(tert.-butyloxycarbonylamino)-piperidin-1-yl]-xanthin
   R_{f}-Wert: 0.50 (Kieselgel, Essigester)
   Massenspektrum (ESI⁺): m/z = 533 [M+H]⁺

### Beispiel III

### 3-Methyl-7-(2-butin-1-yl)-8-brom-xanthin

Zu 30.17 g 3-Methyl-8-brom-xanthin und 27.00 ml Hünigbase in 370 ml N,N-Dimethylformamid werden 17.06 g 1-Brom-2-butin gegeben. Das Reaktionsgemisch wird zwei Stunden bei Raumtemperatur gerührt, dann wird nochmals 1 ml 1-Brom-2-butin nachgesetzt und eine weitere Stunde bei Raumtemperatur gerührt. Zur Aufarbeitung wird das Reaktionsgemisch mit ca. 300 ml Wasser verdünnt. Der entstandene helle Niederschlag wird abgesaugt und mit Wasser nachgewaschen. Der Filterkuchen wird mit wenig Ethanol und Diethylether gewaschen und im Umlufttrockenschrank bei 60°C getrocknet.
Ausbeute: 30.50 g (84 % der Theorie)
R_{f}-Wert: 0.24 (Kieselgel, Methylenchlorid/Methanol = 95:5)
Massenspektrum (ESI⁺): m/z = 297, 299 [M+H]⁺

### Beispiel IV

### 2-Chlormethyl-4-phenylamino-chinazolin

Hergestellt durch Umsetzung von 500 mg 4-Chlor-2-chloromethyl-chinazolin mit 438 mg Anilin in 12 ml Methylenchlorid bei Raumtemperatur.
Ausbeute: 518 mg (82 % der Theorie)
R_{f}-Wert: 0.60 (Kieselgel, Cyclohexan/Essigester = 1:1)
Massenspektrum (ESI⁺): m/z = 270, 272 [M+H]⁺

Analog Beispiel IV wird folgende Verbindung erhalten:
(1) 2-Chlormethyl-4-benzylamino-chinazolin
   R_{f}-Wert: 0.60 (Kieselgel, Cyclohexan/Essigester = 1:1)
   Massenspektrum (ESI⁺): m/z = 284, 286 [M+H]⁺

### Beispiel V

### 1-Brommethyl-4-cyano-isochinolin

Hergestellt durch Bromierung von 1-Methyl-4-cyano-isochinolin mit N-Bromsuccinimid in Gegenwart von Azobisisobutyronitril in Tetrachlorkohlenstoff bei 80°C.
R_{f}-Wert: 0.51 (Kieselgel, Methylenchlorid)
Massenspektrum (EI): m/z = 246, 248 [M]⁺

Analog Beispiel V werden folgende Verbindungen erhalten:
(1) 2-Brommethyl-4-cyano-chinolin
   Massenspektrum (ESI⁺): m/z = 247, 249 [M+H]⁺
(2) 3-Brommethyl-1-cyano-isochinolin
   Massenspektrum (ESI⁺): m/z = 247, 249 [M+H]⁺
(3) 1-Brommethyl-4-(pyridin-2-yl)-isochinolin
   R_{f}-Wert: 0.47 (Kieselgel, Methylenchlorid/Methanol = 9:1)
(4) 2-Brommethyl-4-methoxy-chinolin
   Massenspektrum (ESI⁺): m/z = 252, 254 [M+H]⁺
(5) 3-Brommethyl-[1,5]naphthyridin
   Massenspektrum (ESI⁺): m/z = 223, 225 [M+H]⁺
(6) 2-Brommethyl-5-cyano-chinolin
   R_{f}-Wert: 0.28 (Kieselgel, Petrolether/Essigester = 5:1)
   Massenspektrum (ESI⁺): m/z = 247, 249 [M+H]⁺
(7) 2-Brommethyl-3-cyano-chinolin
   R_{f}-Wert: 0.65 (Kieselgel, Cyclohexan/Essigester = 3:1)
(8) 2-Brommethyl-4-phenyl-pyrimidin
   R_{f}-Wert: 0.88 (Kieselgel, Essigester)
   Massenspektrum (ESI⁺): m/z = 249, 251 [M+H]⁺
(9) 2-Brommethyl-1,4-dicyano-naphthalin
   R_{f}-Wert: 0.48 (Kieselgel, Petrolether/Essigester = 9:1)
   Massenspektrum (EI⁺): m/z = 270, 272 [M]⁺
(10) 2-Brommethyl-6,7-dimethoxy-chinolin
   R_{f}-Wert: 0.70 (Kieselgel, Methylenchlorid/Methanol = 95:5)
   Massenspektrum (ESI⁺): m/z = 282, 284 [M+H]⁺
(11) 2-Brommethyl-4-cyano-chinazolin
   R_{f}-Wert: 0.85 (Kieselgel, Methylenchlorid/Methanol = 99:1)
   Massenspektrum (EI⁺): m/z = 247, 249 [M]⁺
(12) 7-Brommethyl-chinazolin
   R_{f}-Wert: 0.15 (Kieselgel, Methylenchlorid/Methanol = 99:1)
   Massenspektrum (ESI⁺): m/z = 223, 225 [M+H]⁺
(13) 2-Trifluormethyl-4-cyano-benzylbromid
(14) 2-Brommethyl-5-cyano-6-methoxy-pyridin
   Massenspektrum (ESI⁺): m/z = 227, 229 [M+H]⁺
(15) 3-Brommethyl-4-cyano-isochinolin
   R_{f}-Wert: 0.43 (Kieselgel, Petrolether/Essigester = 7:3)
(16) 7-Brommethyl-8-cyano-chinolin
   R_{f}-Wert: 0.25 (Kieselgel, Petrolether/Essigester = 7:3)
   Massenspektrum (ESI⁺): m/z = 247, 249 [M+H]⁺
(17) 2-Brommethyl-8-cyano-chinolin
   R_{f}-Wert: 0.75 (Kieselgel, Methylenchlorid/Methanol = 99:1)
   Massenspektrum (ESI⁺): m/z = 247, 249 [M+H]⁺

### Beispiel VI

### 2-Brom-1-(3-cyclopropyloxy-phenyl)-ethanon

Hergestellt durch Bromierung von 1-(3-Cyclopropyloxy-phenyl)-ethanon mit Phenyltrimethylammoniumtribromid in Methylenchlorid unter Rückfluss.
R_{f}-Wert: 0.75 (Kieselgel, Cyclohexan/Essigester = 3:1)
Massenspektrum (ESI⁺): m/z = 255, 257 [M+H]⁺

Analog Beispiel VI werden folgende Verbindungen erhalten:
(1) 2-Brom-1-(3-cyclopropylmethoxy-phenyl)-ethanon
   R_{f}-Wert: 0.70 (Kieselgel, Cyclohexan/Essigester = 3:1)
(2) 2-Brom-1-(3-cyclobutyloxy-phenyl)-ethanon
   R_{f}-Wert: 0.70 (Kieselgel, Cyclohexan/Essigester = 3:1)

### Beispiel VII

### 1-(3-Cyclopropyloxy-phenyl)-ethanon

Hergestellt durch Umsetzung von 3-Hydroxyacetophenon mit Bromcyclopropan in Gegenwart von Kaliumiodid und Kalium-tert.-butylat in N,N-Dimethylformamid in der Mikrowelle bei 220°C.
R_{f}-Wert: 0.65 (Kieselgel, Cyclohexan/Essigester = 3:1)
Massenspektrum (ESI⁺): m/z = 177 [M+H]⁺

Analog Beispiel VII werden folgende Verbindungen erhalten:
(1) 1-(3-Cyclopropylmethoxy-phenyl)-ethanon
   R_{f}-Wert: 0.70 (Kieselgel, Cyclohexan/Essigester = 3:1)
   Massenspektrum (ESI⁺): m/z = 191 [M+H]⁺
(2) 1-(3-Cyclobutyloxy-phenyl)-ethanon
   R_{f}-Wert: 0.65 (Kieselgel, Cyclohexan/Essigester = 3:1)
   Massenspektrum (ESI⁺): m/z = 191 [M+H]⁺

### Beispiel VIII

### 1-Chlormethyl-2,4-dimethoxy-naphthalin

Hergestellt durch Chlorierung von 1-Hydroxymethyl-2,4-dimethoxy-naphthalin mit Thionylchlorid in Methylenchlorid bei Raumtemperatur.
R_{f}-Wert: 0.78 (Kieselgel, Cyclohexan/Essigester = 1:1)
Massenspektrum (EI): m/z = 236, 238 [M]⁺

### Beispiel IX

### 1-Hydroxymethyl-2,4-dimethoxy-naphthalin

Hergestellt durch Reduktion von 2,4-Dimethoxy-naphthalin-1-carboxaldehyd mit Natriumborhydrid in einem Gemisch aus Dioxan und Wasser (3:1) bei Raumtemperatur.
R_{f}-Wert: 0.48 (Kieselgel, Cyclohexan/Essigester = 1:1)

### Beispiel X

### 1-[(6-Amino-chinolin-2-yl)methyl]-3-methyl-7-(2-butin-1-yl)-8-[(R)-3-(tert.-butyloxycarbonylamino)-piperidin-1-yl]-xanthin

Hergestellt durch Behandlung von 1-[(6-Nitro-chinolin-2-yl)methyl]-3-methyl-7-(2-butin-1-yl)-8-[(*R*)-3-(tert.-butyloxycarbonylamino)-piperidin-1-yl]-xanthin mit Natriumdithionit in einem Gemisch aus Ethanol/Wasser (5:2) bei 55-60°C.
R_{f}-Wert: 0.40 (Kieselgel, Methylenchlorid/Methanol = 95:5)
Massenspektrum (ESI⁺): m/z = 573 [M+H]⁺

### Beispiel XI

### 1-Methyl-4-(pyridin-2-yl)-isochinolin

Hergestellt durch Umsetzung von 4-Brom-1-methyl-isochinolin mit Lithium-Triiisopropoxy-2-pyridinyl-boronat in Gegenwart von Tetrakis(triphenylphosphin)-palladium, Triphenylphosphin, Natriumcarbonat und Kupfer(I)iodid in 1,4-Dioxan unter Rückfluss.
R_{f}-Wert: 0.22 (Kieselgel, Methylenchlorid/Methanol = 95:5)
Massenspektrum (ESI⁺): m/z = 221 [M+H]⁺

### Beispiel XII

### 1-[(8-Amino-chinolin-2-yl)methyl]-3-methyl-7-(2-butin-1-yl)-8-[(R)-3-(tert.-butyloxycarbonylamino)-piperidin-1-yl]-xanthin

Hergestellt durch Behandlung von 1-[(8-Nitro-chinolin-2-yl)methyl]-3-methyl-7-(2-butin-1-yl)-8-[(*R*)-3-(tert.-butyloxycarbonylamino)-piperidin-1-yl]-xanthin mit Eisenpulver in einem Gemisch aus Eisessig, Ethanol und Wasser (2:20:5) unter Rückfluss.
R_{f}-Wert: 0.50 (Kieselgel, Methylenchlorid/Methanol = 95:5)
Massenspektrum (ESI⁺): m/z = 573 [M+H]⁺

### Beispiel XIII

### 1-{2-Oxo-2-[2-(pyridin-3-yl)-phenyl]-ethyl}-3-methyl-7-(2-butin-1-yl)-8-[(R)-3-(tert.-butyloxycarbonylamino)-piperidin-1-yl]-xanthin

Hergestellt durch Umsetzung von 1-[2-Oxo-2-(2-brom-phenyl)-ethyl]-3-methyl-7-(2-butin-1-yl)-8-[(*R*)-3-(tert.-butyloxycarbonylamino)-piperidin-1-yl]-xanthin mit Pyridin-3-boronsäure in Gegenwart von Tetrakis(triphenylphosphin)palladium, Tetra-n-butylammoniumbromid und Natriumcarbonat in einem Gemisch aus Toluol/Ethanol (1:1) bei 105°C.
R_{f}-Wert: 0.55 (Kieselgel, Essigester)
Massenspektrum (ESI⁺): m/z = 612 [M+H]⁺

Analog Beispiel XII wird folgende Verbindung erhalten:
(1) 1-{2-Oxo-2-[2-(pyridin-4-yl)-phenyl]-ethyl}-3-methyl-7-(2-butin-1-yl)-8-[(*R*)-3-(tert.-butyloxycarbonylamino)-piperidin-1-yl]-xanthin
   (Die Umsetzung erfolgt mit 4-(4,4,5,5-Tetramethyl-[1,3,2]dioxaborolan-2-yl)-pyridin.)
   R_{f}-Wert: 0.40 (Kieselgel, Essigester)
   Massenspektrum (ESI⁺): m/z = 612 [M+H]⁺

### Beispiel XIV

### 1-[(4-Ethyl-chinazolin-2-yl)methyl]-3-methyl-7-(2-butin-1-yl)-8-[(R)-3-(tert.-butyloxycarbonylamino)-piperidin-1-yl]-xanthin

Hergestellt durch Behandlung von 1-Cyanomethyl-3-methyl-7-(2-butin-1-yl)-8-[(*R*)-3-(tert.-butyloxycarbonylamino)-piperidin-1-yl]-xanthin mit Kalium-tert.-butylat in Methanol und anschließende Umsetzung des entstandenen Iminoesters mit 2-Aminopropiophenon in Gegenwart von Eisessig.
R_{f}-Wert: 0.60 (Kieselgel, Essigester)
Massenspektrum (ESI⁺): m/z = 587 [M+H]⁺

Analog Beispiel XIV wird folgende Verbindung erhalten:
(1) 1-[(4-Cyclopropyl-chinazolin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.70 (Kieselgel, Essigester)
   Massenspektrum (ESI⁺): m/z = 599 [M+H]⁺

### Beispiel XV

### 2-Chlormethyl-3-cyano-4-methyl-chinolin

Hergestellt durch Umsetzung von 3-Cyano-2,4-dimethyl-1-oxy-chinolin
mit Benzosulfonsäurechlorid in Toluol bei 80°C.
R_{f}-Wert: 0.55 (Kieselgel, Cyclohexan/Essigester = 2:1)
Massenspektrum (ESI⁺): m/z = 217, 219 [M+H]⁺

### Beispiel XVI

### 3-Cyano-2,4-dimethyl-1-oxy-chinolin

Hergestellt durch Behandlung von 3-Cyano-2,4-dimethyl-chinolin mit wässriger Wasserstoffperoxidlösung (35 %) in Eisessig bei 60°C.
R_{f}-Wert: 0.35 (Kieselgel, Essigester)
Massenspektrum (ESI⁺): m/z = 199 [M+H]⁺

### Beispiel XVII

### 2-Chlormethyl-4,5-dimethyl-chinazolin

Hergestellt durch Umsetzung von 1-(2-Amino-6-methyl-phenyl)-ethanon mit Chloracetonitril in Dioxan unter Einleitung von Chlorwasserstoff bei 30-38°C. Massenspektrum (ESI⁺): m/z = 207, 209 [M+H]⁺

### Beispiel XVIII

### 1-[(2-Methyl-chinazolin-4-yl)methyl]-3-methyl-7-(2-butin-1-yl)-8-[(R)-3-(tert.-butyloxycarbonylamino)-piperidin-1-yl]-xanthin

Hergestellt durch Umsetzung von 1-[2-(2-Acetylamino-phenyl)-2-oxo-ethyl]-3-methyl-7-(2-butin-1-yl)-8-[(R)-3-(tert.-butyloxycarbonylamino)-piperidin-1-yl]-xanthin mit ethanolischem Ammoniak (6 M) und Ammoniumchlorid im Autoklaven bei 150°C.
R_{f}-Wert: 0.35 (Kieselgel, Essigester)
Massenspektrum (ESI⁺): m/z = 573 [M+H]⁺

### Beispiel XIX

### 1-[2-(2-Acetylamino-phenyl)-2-oxo-ethyl]-3-methyl-7-(2-butin-1-yl)-8-[(R)-3-(tert.-butyloxycarbonylamino)-piperidin-1-yl]-xanthin

Hergestellt durch Umsetzung von 1-[2-(2-Amino-phenyl)-2-oxo-ethyl]-3-methyl-7-(2-butin-1-yl)-8-[(*R*)-3-(tert.-butyloxycarbonylamino)-piperidin-1-yl]-xanthin mit Acetylchlorid in Gegenwart von Pyridin in Methylenchlorid bei Raumtemperatur.
R_{f}-Wert: 0.79 (Kieselgel, Essigester)
Massenspektrum (ESI⁺): m/z = 592 [M+H]⁺

### Beispiel XX

### 1-[2-(2-Amino-phenyl)-2-oxo-ethyl]-3-methyl-7-(2-butin-1-yl)-8-[(R)-3-(tert.-butyloxycarbonylamino)-piperidin-1-yl]-xanthin

Hergestellt durch Reduktion von 1-[2-(2-Nitro-phenyl)-2-oxo-ethyl]-3-methyl-7-(2-butin-1-yl)-8-[(*R*)-3-(tert.-butyloxycarbonylamino)-piperidin-1-yl]-xanthin mit Zinn(II)-chlorid-dihydrat in Tetrahydrofuran bei Raumtemperatur.
R_{f}-Wert: 0.85 (Kieselgel, Essigester)
Massenspektrum (ESI⁺): m/z = 550 [M+H]⁺

### Beispiel XXI

### 1-[(Furan-3-yl)methyl]-3-methyl-7-(2-butin-1-yl)-8-[(R)-3-(tert.-butyloxycarbonylamino)-piperidin-1-yl]-xanthin

Ein Gemisch aus 300 mg 3-Methyl-7-(2-butin-1-yl)-8-[(R)-3-(tert.-butyloxycarbonylamino)-piperidin-1-yl]-xanthin, 95 µl Furan-3-yl-methanol, 302 mg Triphenylphosphin und 226 µl Diisopropylazodicarboxylat in 4 ml Tetrahydrofuran wird über Nacht bei Raumtemperatur gerührt. Zur Aufarbeitung wird das Reaktionsgemisch mit gesättigter Kaliumcarbonat-Lösung versetzt und mit Essigester extrahiert. Die vereinigten organischen Phasen werden über Magnesiumsulfat getrocknet und eingeengt. Der Kolbenrückstand wird über eine Kieselgelsäule mit Cyclohexan/Essigester (1:1 auf 3:7) chromatographiert.
Ausbeute: 330 mg (92 % der Theorie)
Massenspektrum (ESI⁺): m/z = 497 [M+H]⁺

Analog Beispiel XXI werden folgende Verbindungen erhalten:
(1) 1-[(5-Methyl-furan-2-yl)methyl]-3-methyl-7-(2-butin-1-yl)-8-brom-xanthin Massenspektrum (ESI⁺): m/z = 391, 393 [M+H]⁺
(2) 1-[(5-Brom-furan-2-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 575, 577 [M+H]⁺

### Beispiel XXII

### 1-[(5-Cyano-furan-2-yl)methyl]-3-methyl-7-(2-butin-1-yl)-8-[(R)-3-(tert.-butyloxycarbonylamino)-piperidin-1-yl]-xanthin

Hergestellt durch Umsetzung von 1-[(5-Formyl-furan-2-yl)methyl]-3-methyl-7-(2-butin-1-yl)-8-[(*R*)-3-(tert.-butyloxycarbonylamino)-piperidin-1-yl]-xanthin mit Hydroxylamin-O-sulfonsäure und Pyridin in Toluol unter Rückfluss.

### Beispiel XXIII

### 5-(Methansulfonyloxymethyl)-2-furan-carboxaldehyd

Hergestellt durch Umsetzung von 5-(Hydroxymethyl)-2-furan-carboxaldehyd mit Methansulfonsäurechlorid in Gegenwart von Triethylamin in Methylenchlorid bei Raumtemperatur. Das Rohprodukt wird ohne weitere Reingung weiter umgesetzt.

### Beispiel XXIV

### 2-Chlormethyl-3-cyano-pyridin

Hergestellt aus 2-(Hydroxymethyl)-nicotinamid durch Umsetzung mit Thionylchlorid in Acetonitril und anschliessende Dehydratisierung des so erhaltenen 2-(Chlormethyl)-nicotinamids mit Trifluoressigsäureanhydrid in Gegenwart von Triethylamin in Methylenchlorid.

Alternativ wird die Verbindung auch in einem Schritt durch Erhitzen von 2-(Hydroxymethyl)-nicotinamid mit Phosphoroxychlorid unter Rückfluß erhalten.
R_{f}-Wert: 0.85 (Kieselgel, Methylenchlorid/Methanol = 9:1)
Massenspektrum (ESI⁺): m/z = 153, 155 [M+H]⁺

### Beispiel XXV

### 8-Cyano-7-methyl-chinolin

Hergestellt durch Umsetzung von 8-Brom-7-methyl-chinolin mit Zinkcyanid in Gegenwart von Tetrakis(triphenylphosphin)palladium in N-Methylpyrrolidinon unter Schutzgasatmosphäre bei 100-105°C.
R_{f}-Wert: 0.35 (Kieselgel, Petrolether/Essigester = 7:3)
Massenspektrum (ESI⁺): m/z = 169 [M+H]⁺

### Beispiel XXVI

### 2-Methyl-8-cyano-chinolin

Hergestellt durch Umsetzung von 2-Methyl-8-brom-chinolin mit Kupfer(I)cyanid in N-Methylpyrrolidinon unter Schutzgasatmosphäre bei 180°C.
R_{f}-Wert: 0.40 (Kieselgel, Petrolether/Essigester = 7:3)
Massenspektrum (ESI⁺): m/z = 169 [M+H]⁺

### Herstellung der Endverbindungen

### Beispiel 1

### 1-[(4-Phenylamino-chinazolin-2-yl)methyl]-3-methyl-7-(2-butin-1-yl)-8-(3-aminopiperidin-1-yl)-xanthin

Ein Gemisch aus 400 mg 1-[(4-Phenylamino-chinazolin-2-yl)methyl]-3-methyl-7-(2-butin-1-yl)-8-[3-(tert.-butyloxycarbonylamino)-piperidin-1-yl]-xanthin in 10 ml Methylenchlorid wir mit 2 ml isopropanolischer Salzsäure (5-6 M) versetzt und drei Stunden bei Raumtemperatur gerührt. Anschließend wird das Reaktionsgemisch mit Methylenchlorid verdünnt, mit Eiswasser versetzt und mit 3 M Kaliumcarbonatlösung alkalisch gestellt. Die wässrige Phase wird mit Methylenchlorid extrahiert. Die vereinigten Extrakte werden mit Wasser gewaschen, über Magnesiumsulfat getrocknet und eingeengt. Der Kolbenrückstand wird mit Diethylether verrührt, abgesaugt, mit Diethylether nachgewaschen und im Vakuum getrocknet.
Ausbeute: 274 mg (81 % der Theorie)
R_{f}-Wert: 0.50 (Kieselgel, Methylenchlorid/Methanol/konz. wässriges Ammoniak = 90:10:0.1)
Massenspektrum (ESI⁺): m/z = 550 [M+H]⁺

Analog Beispiel 1 werden folgende Verbindungen erhalten:
(1) 1-[(4-Benzylamino-chinazolin-2-yl)methyl]-3-methyl-7-(2-butin-1-yl)-8-(3-aminopiperidin-1-yl)-xanthin
   R_{f}-Wert: 0.43 (Kieselgel, Methylenchlorid/Methanol/konz. wässriges Ammoniak = 90:10:0.1)
   Massenspektrum (ESI⁺): m/z = 564 [M+H]⁺
(2) 1-[(2-Methyl-chinolin-4-yl)methyl]-3-methyl-7-(2-butin-1-yl)-8-((*R*)-3-aminopiperidin-1-yl)-xanthin
   Massenspektrum (ESI⁺): m/z = 472 [M+H]⁺
(3) 1-[(3-Cyano-naphthalin-1-yl)methyl]-3-methyl-7-(2-butin-1-yl)-8-((*R*)-3-aminopiperidin-1-yl)-xanthin
   R_{f}-Wert: 0.55 (Kieselgel, Methylenchlorid/Methanol/konz. wässriges Ammoniak = 90:10:1)
   Massenspektrum (ESI⁺): m/z = 482 [M+H]⁺
(4) 1-[(2-Phenyl-chinazolin-4-yl)methyl]-3-methyl-7-(2-butin-1-yl)-8-((*R*)-3-aminopiperidin-1-yl)-xanthin
   R_{f}-Wert: 0.45 (Reversed Phase DC-Fertigplatte (E. Merck), Acetonitril/Wasser/ Trifluoressigsäure = 50:50:1)
   Massenspektrum (ESI⁺): m/z = 535 [M+H]⁺
(5) 1-[(4-Cyano-isochinolin-1-yl)methyl]-3-methyl-7-(2-butin-1-yl)-8-((*R*)-3-aminopiperidin-1-yl)-xanthin
   R_{f}-Wert: 0.15 (Kieselgel, Methylenchlorid/Methanol/konz. wässriges Ammoniak = 90:10:1)
   Massenspektrum (ESI⁺): m/z = 483 [M+H]⁺
(6) 1-[(4-Cyano-chinolin-2-yl)methyl]-3-methyl-7-(2-butin-1-yl)-8-((*R*)-3-aminopiperidin-1-yl)-xanthin
   Massenspektrum (ESI⁺): m/z = 483 [M+H]⁺
(7) 1-[2-(3-Cyclopropyloxy-phenyl)-2-oxo-ethyl]-3-methyl-7-(2-butin-1-yl)-8-((*R*)-3-amino-piperidin-1-yl)-xanthin
   R_{f}-Wert: 0.45 (Reversed Phase DC-Fertigplatte (E. Merck), Acetonitril/Wasser/ Trifluoressigsäure = 50:50:1)
   Massenspektrum (ESI⁺): m/z = 491 [M+H]⁺
(8) 1-[2-(3-Cyclopropylmethoxy-phenyl)-2-oxo-ethyl]-3-methyl-7-(2-butin-1-yl)-8-((*R*)-3-amino-piperidin-1-yl)-xanthin
   R_{f}-Wert: 0.35 (Reversed Phase DC-Fertigplatte (E. Merck), Acetonitril/Wasser/ Trifluoressigsäure = 50:50:1)
   Massenspektrum (ESI⁺): m/z = 505 [M+H]⁺
(9) 1-[2-(3-Cyclobutyloxy-phenyl)-2-oxo-ethyl]-3-methyl-7-(2-butin-1-yl)-8-((*R*)-3-amino-piperidin-1-yl)-xanthin
   R_{f}-Wert: 0.40 (Reversed Phase DC-Fertigplatte (E. Merck), Acetonitril/Wasser/ Trifluoressigsäure = 50:50:1)
   Massenspektrum (ESI⁺): m/z = 505 [M+H]⁺
(10) 1-[(1-Cyano-isochinolin-3-yl)methyl]-3-methyl-7-(2-butin-1-yl)-8-((*R*)-3-amino-piperidin-1-yl)-xanthin
   Massenspektrum (ESI⁺): m/z = 483 [M+H]⁺
(11) 1-[(2,4-Methoxy-naphthalin-1-yl)methyl]-3-methyl-7-(2-butin-1-yl)-8-((R)-3-amino-piperidin-1-yl)-xanthin
   R_{f}-Wert: 0.55 (Kieselgel, Methylenchlorid/Methanol/konz. wässriges Ammoniak = 90:10:0.1)
   Massenspektrum (ESI⁺): m/z = 517 [M+H]⁺
(12) 1-[(2,3-Dimethyl-chinoxalin-6-yl)methyl]-3-methyl-7-(2-butin-1-yl)-8-((R)-3-amino-piperidin-1-yl)-xanthin
   R_{f}-Wert: 0.48 (Kieselgel, Methylenchlorid/Methanol/konz. wässriges Ammoniak = 90:10:0.1)
   Massenspektrum (ESI⁺): m/z = 487 [M+H]⁺
(13) 1-[(6-Amino-chinolin-2-yl)methyl]-3-methyl-7-(2-butin-1-yl)-8-((R)-3-amino-piperidin-1-yl)-xanthin
   R_{f}-Wert: 0.40 (Kieselgel, Methylenchlorid/Methanol/konz. wässriges Ammoniak = 90:10:0.1)
   Massenspektrum (ESI⁺): m/z = 473 [M+H]⁺
(14) 1-[(Chinoxalin-5-yl)methyl]-3-methyl-7-(2-butin-1-yl)-8-((R)-3-amino-piperidin-1-yl)-xanthin-hydrochlorid
   Massenspektrum (ESI⁺): m/z = 459 [M+H]⁺
(15) 1-[(6-Methoxy-chinolin-2-yl)methyl]-3-methyl-7-(2-butin-1-yl)-8-((*R*)-3-amino-piperidin-1-yl)-xanthin
   R_{f}-Wert: 0.50 (Kieselgel, Methylenchlorid/Methanol/konz. wässriges Ammoniak = 90:10:0.1)
   Massenspektrum (ESI⁺): m/z = 488 [M+H]⁺
(16) 1-[(6-Phenyl-pyridin-2-yl)methyl]-3-methyl-7-(2-butin-1-yl)-8-((*R*)-3-amino-piperidin-1-yl)-xanthin
   R_{f}-Wert: 0.37 (Kieselgel, Methylenchlorid/Methanol/konz. wässriges Ammoniak = 90:10:1)
   Massenspektrum (ESI⁺): m/z = 484 [M+H]⁺
(17) 1-{[(4-(Pyridin-2-yl)-isochinolin-1-yl]methyl}-3-methyl-7-(2-butin-1-yl)-8-((*R*)-3-amino-piperidin-1-yl)-xanthin
   R_{f}-Wert: 0.37 (Kieselgel, Methylenchlorid/Methanol/konz. wässriges Ammoniak = 80:20:1)
   Massenspektrum (ESI⁺): m/z = 535 [M+H]⁺
(18) 1-[(7-Fluor-chinolin-2-yl)methyl]-3-methyl-7-(2-butin-1-yl)-8-((R)-3-amino-piperidin-1-yl)-xanthin
   R_{f}-Wert: 0.58 (Kieselgel, Methylenchlorid/Methanol/konz. wässriges Ammoniak = 90:10:1)
   Massenspektrum (ESI⁺): m/z = 476 [M+H]⁺
(19) 1-[(8-Amino-chinolin-2-yl)methyl]-3-methyl-7-(2-butin-1-yl)-8-((R)-3-amino-piperidin-1-yl)-xanthin
   R_{f}-Wert: 0.50 (Kieselgel, Methylenchlorid/Methanol/konz. wässriges Ammoniak = 90:10:1)
   Massenspektrum (ESI⁺): m/z = 473 [M+H]⁺
(20) 1-[(6-Fluor-chinolin-2-yl)methyl]-3-methyl-7-(2-butin-1-yl)-8-((R)-3-amino-piperidin-1-yl)-xanthin
   R_{f}-Wert: 0.50 (Kieselgel, Methylenchlorid/Methanol/konz. wässriges Ammoniak = 90:10:1)
   Massenspektrum (ESI⁺): m/z = 476 [M+H]⁺
(21) 1-{2-Oxo-2-[2-(pyridin-3-yl)-phenyl]-ethyl}-3-methyl-7-(2-butin-1-yl)-8-((*R*)-3-amino-piperidin-1-yl)-xanthin
   R_{f}-Wert: 0.55 (Reversed Phase DC-Fertigplatte (E. Merck), Acetonitril/Wasser/ Trifluoressigsäure = 50:50:1)
   Massenspektrum (ESI⁺): m/z = 512 [M+H]⁺
(22) 1-[(4-Ethyl-chinazolin-2-yl)methyl]-3-methyl-7-(2-butin-1-yl)-8-((*R*)-3-amino-piperidin-1-yl)-xanthin
   R_{f}-Wert: 0.45 (Kieselgel, Methylenchlorid/Methanol/konz. wässriges Ammoniak = 90:10:0.1)
   Massenspektrum (ESI⁺): m/z = 487 [M+H]⁺
(23) 1-[(4-Cyclopropyl-chinazolin-2-yl)methyl]-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.45 (Kieselgel, Methylenchlorid/Methanol/konz. wässriges Ammoniak = 90:10:0.1)
   Massenspektrum (ESI⁺): m/z = 499 [M+H]⁺
(24) 1-[(4-Methoxy-chinolin-2-yl)methyl]-3-methyl-7-(2-butin-1-yl)-8-((*R*)-3-amino-piperidin-1-yl)-xanthin
   Massenspektrum (ESI⁺): m/z = 488 [M+H]⁺
(25) 1-[(2-Phenyl-pyrimidin-4-yl)methyl]-3-methyl-7-(2-butin-1-yl)-8-((*R*)-3-amino-piperidin-1-yl)-xanthin
   R_{f}-Wert: 0.50 (Kieselgel, Methylenchlorid/Methanol/konz. wässriges Ammoniak = 90:10:1)
   Massenspektrum (ESI⁺): m/z = 485 [M+H]⁺
(26) 1-[([1,5]Naphthyridin-3-yl)methyl]-3-methyl-7-(2-butin-1-yl)-8-((*R*)-3-amino-piperidin-1-yl)-xanthin
   R_{f}-Wert: 0.52 (Kieselgel, Methylenchlorid/Methanol/konz. wässriges Ammoniak = 90:10:1)
   Massenspektrum (ESI⁺): m/z = 459 [M+H]⁺
(27) 1-[(3-Cyano-4-methyl-chinolin-2-yl)methyl]-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.50 (Reversed Phase DC-Fertigplatte (E. Merck), Acetonitril/Wasser/ Trifluoressigsäure = 50:50:1)
   Massenspektrum (ESI⁺): m/z = 497 [M+H]⁺
(28) 1-[(4,5-Dimethyl-chinazolin-2-yl)methyl]-3-methyl-7-(2-butin-1-yl)-8-((*R*)-3-amino-piperidin-1-yl)-xanthin
   (BOC-Spaltung erfolgt mit Trifluoressigsäure)
   Massenspektrum (ESI⁺): m/z = 487 [M+H]⁺
(29) 1-[(5-Cyano-chinolin-2-yl)methyl]-3-methyl-7-(2-butin-1-yl)-8-((*R*)-3-amino-piperidin-1-yl)-xanthin
   R_{f}-Wert: 0.50 (Kieselgel, Methylenchlorid/Methanol/konz. wässriges Ammoniak = 90:10:1)
   Massenspektrum (ESI⁺): m/z = 483 [M+H]⁺
(30) 1-[(3-Cyano-chinolin-2-yl)methyl]-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.50 (Reversed Phase DC-Fertigplatte (E. Merck), Acetonitril/Wasser/ Trifluoressigsäure = 50:50:1)
   Massenspektrum (ESI⁺): m/z = 483 [M+H]⁺
(31) 1-[(4-Phenyl-pyrimidin-2-yl)methyl]-3-methyl-7-(2-butin-1-yl)-8-((*R*)-3-amino-piperidin-1-yl)-xanthin
   R_{f}-Wert: 0.50 (Kieselgel, Methylenchlorid/Methanol/konz. wässriges Ammoniak = 90:10:1)
   Massenspektrum (ESI⁺): m/z = 485 [M+H]⁺
(32) 1-[(2-Methyl-chinazolin-4-yl)methyl]-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.38 (Kieselgel, Methylenchlorid/Methanol/konz. wässriges Ammoniak = 90:10:1)
   Massenspektrum (ESI⁺): m/z = 473 [M+H]⁺
(33) 1-[(1,4-Dicyano-naphthalin-2-yl)methyl]-3-methyl-7-(2-butin-1-yl)-8-((*R*)-3-amino-piperidin-1-yl)-xanthin-hydrochlorid
   R_{f}-Wert: 0.86 (Kieselgel, Methylenchlorid/Methanol/konz. wässriges Ammoniak = 90:10:1)
   Massenspektrum (ESI⁺): m/z = 507 [M+H]⁺
(34) 1-{2-Oxo-2-[2-(pyridin-4-yl)-phenyl]-ethyl}-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.55 (Reversed Phase DC-Fertigplatte (E. Merck), Acetonitril/Wasser/ Trifluoressigsäure = 50:50:1)
   Massenspektrum (ESI⁺): m/z = 512 [M+H]⁺
(35) 1-[(6,7-Dimethoxy-chinolin-2-yl)methyl]-3-methyl-7-(2-butin-1-yl)-8-((*R*)-3-amino-piperidin-1-yl)-xanthin
   R_{f}-Wert: 0.45 (Kieselgel, Methylenchlorid/Methanol/konz. wässriges Ammoniak = 90:10:1)
   Massenspektrum (ESI⁺): m/z = 518 [M+H]⁺
(36) 1-[(Chinazolin-6-yl)methyl]-3-methyl-7-(2-butin-1-yl)-8-((*R*)-3-amino-piperidin-1-yl)-xanthin-hydrochlorid
   R_{f}-Wert: 0.50 (Kieselgel, Methylenchlorid/Methanol/konz. wässriges Ammoniak = 90:10:0.1)
   Massenspektrum (ESI⁺): m/z = 459 [M+H]⁺
(37) 1-[(4-Cyano-chinazolin-2-yl)methyl]-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.50 (Kieselgel, Methylenchlorid/Methanol/konz. wässriges Ammoniak = 90:10:1)
   Massenspektrum (ESI⁺): m/z = 484 [M+H]⁺
(38) 1-[(Chinazolin-7-yl)methyl]-3-methyl-7-(2-butin-1-yl)-8-((*R*)-3-amino-piperidin-1-yl)-xanthin
   R_{f}-Wert: 0.43 (Kieselgel, Methylenchlorid/Methanol/konz. wässriges Ammoniak = 90:10:0.1)
   Massenspektrum (ESI⁺): m/z = 459 [M+H]⁺
(39) 1-(2-Cyano-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.35 (Kieselgel, Methylenchlorid/Methanol/konz. wässriges Ammoniak = 90:10:1)
   Massenspektrum (ESI⁺): m/z = 432 [M+H]⁺
(40) 1-(3-Cyano-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.40 (Kieselgel, Methylenchlorid/Methanol/konz. wässriges Ammoniak = 90:10:1)
   Massenspektrum (ESI⁺): m/z = 432 [M+H]⁺
(41) 1-(4-Cyano-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.31 (Kieselgel, Methylenchlorid/Methanol/konz. wässriges Ammoniak = 90:10:1)
   Massenspektrum (ESI⁺): m/z = 432 [M+H]⁺
(42) 1-[(Pyridin-2-yl)methyl]-3-methyl-7-(2-butin-1-yl)-8-((*R*)-3-amino-piperidin-1-yl)-xanthin
   R_{f}-Wert: 0.52 (Kieselgel, Methylenchlorid/Methanol/konz. wässriges Ammoniak = 90:10:1)
   Massenspektrum (ESI⁺): m/z = 408 [M+H]⁺
(43) 1-Benzyl-3-methyl-7-(2-butin-1-yl)-8-((*R*)-3-amino-piperidin-1-yl)-xanthin
   (BOC-Spaltung erfolgt mit Trifluoressigsäure)
   Schmelzpunkt: 207-209°C
   Massenspektrum (ESI⁺): m/z = 407 [M+H]⁺
(44) 1-(4-Methoxy-benzyl)-3-methyl-7-(2-butin-1-yl)-8-((*R*)-3-amino-piperidin-1-yl)-xanthin-hydrochlorid
   R_{f}-Wert: 0.50 (Kieselgel, Methylenchlorid/Methanol/konz. wässriges Ammoniak = 90:10:0.1)
   Massenspektrum (ESI⁺): m/z = 437 [M+H]⁺
(45) 1-(2-Chlor-benzyl)-3-methyl-7-(2-butin-1-yl)-8-((*R*)-3-amino-piperidin-1-yl)-xanthin
   R_{f}-Wert: 0.50 (Kieselgel, Methylenchlorid/Methanol/konz. wässriges Ammoniak = 90:10:0.1)
   Massenspektrum (ESI⁺): m/z = 441, 443 [M+H]⁺
(46) 1-(2,6-Dicyano-benzyl)-3-methyl-7-(2-butin-1-yl)-8-((*R*)-3-amino-piperidin-1-yl)-xanthin x Trifluoressigsäure
   (BOC-Spaltung erfolgt mit Trifluoressigsäure)
   Massenspektrum (ESI⁺): m/z = 457 [M+H]⁺
(47) 1-(2-Cyano-4-brom-benzyl)-3-methyl-7-(2-butin-1-yl)-8-((*R*)-3-amino-piperidin-1-yl)-xanthin x Trifluoressigsäure
   (BOC-Spaltung erfolgt mit Trifluoressigsäure)
   Massenspektrum (ESI⁺): m/z = 510, 512 [M+H]⁺
(48) 1-(3-Fluor-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.45 (Kieselgel, Methylenchlorid/Methanol/konz. wässriges Ammoniak = 90:10:0.1)
   Massenspektrum (ESI⁺): m/z = 425 [M+H]⁺
(49) 1-(3,5-Dimethoxy-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.45 (Kieselgel, Methylenchlorid/Methanol/konz. wässriges Ammoniak = 90:10:0.1)
   Massenspektrum (ESI⁺): m/z = 467 [M+H]⁺
(50) 1-(2-Fluor-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.45 (Kieselgel, Methylenchlorid/Methanol/konz. wässriges Ammoniak = 90:10:0.1)
   Massenspektrum (ESI⁺): m/z = 425 [M+H]⁺
(51) 1-[(6-Cyano-pyridin-2-yl)methyl]-3-methyl-7-(2-butin-1-yl)-8-((*R*)-3-amino-piperidin-1-yl)-xanthin
   (BOC-Spaltung erfolgt mit Trifluoressigsäure)
   Massenspektrum (ESI⁺): m/z = 433 [M+H]⁺
(52) 1-[(3-Cyano-pyridin-2-yl)methyl]-3-methyl-7-(2-butin-1-yl)-8-((*R*)-3-amino-piperidin-1-yl)-xanthin
   (BOC-Spaltung erfolgt mit Trifluoressigsäure)
   Massenspektrum (ESI⁺): m/z = 433 [M+H]⁺
(53) 1-(2-Cyano-3-chlor-benzyl)-3-methyl-7-(2-butin-1-yl)-8-((*R*)-3-amino-piperidin-1-yl)-xanthin x Trifluoressigsäure
   (BOC-Spaltung erfolgt mit Trifluoressigsäure)
   Massenspektrum (ESI⁺): m/z = 466, 468 [M+H]⁺
(54) 1-(4-Fluor-benzyl)-3-methyl-7-(2-butin-1-yl)-8-((*R*)-3-amino-piperidin-1-yl)-xanthin
   (BOC-Spaltung erfolgt mit Trifluoressigsäure)
   Massenspektrum (ESI⁺): m/z = 425 [M+H]⁺
(55) 1-(4-Chlor-benzyl)-3-methyl-7-(2-butin-1-yl)-8-((*R*)-3-amino-piperidin-1-yl)-xanthin
   (BOC-Spaltung erfolgt mit Trifluoressigsäure)
   Massenspektrum (ESI⁺): m/z = 441, 443 [M+H]⁺
(56) 1-(2-Cyano-4-fluor-benzyl)-3-methyl-7-(2-butin-1-yl)-8-((*R*)-3-amino-piperidin-1-yl)-xanthin x Trifluoressigsäure
   (BOC-Spaltung erfolgt mit Trifluoressigsäure)
   Massenspektrum (ESI⁺): m/z = 450 [M+H]⁺
(57) 1-(3-Cyano-4-fluor-benzyl)-3-methyl-7-(2-butin-1-yl)-8-((*R*)-3-amino-piperidin-1-yl)-xanthin x Trifluoressigsäure
   (BOC-Spaltung erfolgt mit Trifluoressigsäure)
   Massenspektrum (ESI⁺): m/z = 450 [M+H]⁺
(58) 1-(2-Chlor-4-cyano-benzyl)-3-methyl-7-(2-butin-1-yl)-8-((*R*)-3-amino-piperidin-1-yl)-xanthin x Trifluoressigsäure
   (BOC-Spaltung erfolgt mit Trifluoressigsäure)
   Massenspektrum (ESI⁺): m/z = 466, 468 [M+H]⁺
(59) 1-[(5-Methoxycarbonyl-furan-2-yl)methyl]-3-methyl-7-(2-butin-1-yl)-8-((*R*)-3-amino-piperidin-1-yl)-xanthin x Trifluoressigsäure
   (BOC-Spaltung erfolgt mit Trifluoressigsäure)
   Massenspektrum (ESI⁺): m/z = 455 [M+H]⁺
(60) 1-(2-Trifluormethyl-4-cyano-benzyl)-3-methyl-7-(2-butin-1-yl)-8-((*R*)-3-amino-piperidin-1-yl)-xanthinx x Trifluoressigsäure
   (BOC-Spaltung erfolgt mit Trifluoressigsäure)
   Massenspektrum (ESI⁺): m/z = 500 [M+H]⁺
(61) 1-(3,5-Dicyano-benzyl)-3-methyl-7-(2-butin-1-yl)-8-((*R*)-3-amino-piperidin-1-yl)-xanthin
   (BOC-Spaltung erfolgt mit Trifluoressigsäure)
   Massenspektrum (ESI⁺): m/z = 457 [M+H]⁺
(62) 1-(3-Nitro-4-cyano-benzyl)-3-methyl-7-(2-butin-1-yl)-8-((*R*)-3-amino-piperidin-1-yl)-xanthin x Trifluoressigsäure
   (BOC-Spaltung erfolgt mit Trifluoressigsäure)
   Massenspektrum (ESI⁺): m/z = 477 [M+H]⁺
(63) 1-[(2-Cyano-pyridin-3-yl)methyl]-3-methyl-7-(2-butin-1-yl)-8-(*(*R)-3-amino-piperidin-1-yl)-xanthin
   (BOC-Spaltung erfolgt mit Trifluoressigsäure)
   R_{f}-Wert: 0.50 (Reversed Phase DC-Fertigplatte (E. Merck), Acetonitril/Wasser/ Trifluoressigsäure = 50:50:1)
   Massenspektrum (ESI⁺): m/z = 433 [M+H]⁺
(64) 1-(2-Cyano-4-methoxy-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.50 (Reversed Phase DC-Fertigplatte (E. Merck), Acetonitril/Wasser/ Trifluoressigsäure = 50:50:1)
   Massenspektrum (ESI⁺): m/z = 462 [M+H]⁺
(65) 1-(2-Cyano-5-methoxy-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.45 (Reversed Phase DC-Fertigplatte (E. Merck), Acetonitril/Wasser/ Trifluoressigsäure = 50:50:1)
   Massenspektrum (ESI⁺): m/z = 462 [M+H]⁺
(66) 1-(3-Methoxy-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.45 (Kieselgel, Methylenchlorid/Methanol/konz. wässriges Ammoniak = 90:10:0.1)
   Massenspektrum (ESI⁺): m/z = 437 [M+H]⁺
(67) 1-(3-Trifluormethyl-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.60 (Kieselgel, Methylenchlorid/Methanol/konz. wässriges Ammoniak = 90:10:0.1)
   Massenspektrum (ESI⁺): m/z = 475 [M+H]⁺
(68) 1-(3,4-Dimethoxy-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.50 (Kieselgel, Methylenchlorid/Methanol/konz. wässriges Ammoniak = 90:10:0.1)
   Massenspektrum (ESI⁺): m/z = 467 [M+H]⁺
(69) 1-(3-Chlor-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.45 (Kieselgel, Methylenchlorid/Methanol/konz. wässriges Ammoniak = 90:10:0.1)
   Massenspektrum (ESI⁺): m/z = 441, 443 [M+H]⁺
(70) 1-(4-Trifluormethyl-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.45 (Kieselgel, Methylenchlorid/Methanol/konz. wässriges Ammoniak = 90:10:0.1)
   Massenspektrum (ESI⁺): m/z = 475 [M+H]⁺
(71) 1-[([2,2']Bipyridinyl-6-yl)methyl]-3-methyl-7-(2-butin-1-yl)-8-((*R*)-3-amino-piperidin-1-yl)-xanthin
   R_{f}-Wert: 0.50 (Kieselgel, Methylenchlorid/Methanol/konz. wässriges Ammoniak = 90:10:1)
   Massenspektrum (ESI⁺): m/z = 485 [M+H]⁺
(72) 1-(3,4-Dimethoxy-6-fluor-benzyl)-3-methyl-7-(2-butin-1-yl)-8-((*R*)-3-amino-piperidin-1-yl)-xanthin
   R_{f}-Wert: 0.50 (Kieselgel, Methylenchlorid/Methanol/konz. wässriges Ammoniak = 90:10:0.1)
   Massenspektrum (ESI⁺): m/z = 485 [M+H]⁺
(73) 1-[(6-Fluor-pyridin-2-yl)methyl]-3-methyl-7-(2-butin-1-yl)-8-((*R*)-3-amino-piperidin-1-yl)-xanthin
   R_{f}-Wert: 0.41 (Kieselgel, Methylenchlorid/Methanol/konz. wässriges Ammoniak = 90:10:1)
   Massenspektrum (ESI⁺): m/z = 426 [M+H]⁺
(74) 1-[(5-Cyano-6-methoxy-pyridin-2-yl)methyl]-3-methyl-7-(2-butin-1-yl)-8-((*R*)-3-amino-piperidin-1-yl)-xanthin
   R_{f}-Wert: 0.40 (Kieselgel, Methylenchlorid/Methanol/konz. wässriges Ammoniak = 90:10:1)
   Massenspektrum (ESI⁺): m/z = 463 [M+H]⁺
(75) 1-(2,6-Difluor-benzyl)-3-methyl-7-(2-butin-1-yl)-8-((*R*)-3-amino-piperidin-1-yl)-xanthin
   R_{f}-Wert: 0.41 (Kieselgel, Methylenchlorid/Methanol/konz. wässriges Ammoniak = 90:10:1)
   Massenspektrum (ESI⁺): m/z = 443 [M+H]⁺
(76) 1-(3-Trifluormethoxy-benzyl)-3-methyl-7-(2-butin-1-yl)-8-((*R*)-3-amino-piperidin-1-yl)-xanthin
   R_{f}-Wert: 0.36 (Kieselgel, Methylenchlorid/Methanol/konz. wässriges Ammoniak = 90:10:1)
   Massenspektrum (ESI⁺): m/z = 491 [M+H]⁺
(77) 1-(4-Trifluormethoxy-benzyl)-3-methyl-7-(2-butin-1-yl)-8-((*R*)-3-amino-piperidin-1-yl)-xanthin
   R_{f}-Wert: 0.38 (Kieselgel, Methylenchlorid/Methanol/konz. wässriges Ammoniak = 90:10:1)
   Massenspektrum (ESI⁺): m/z = 491 [M+H]⁺
(78) 1-[(2-Cyano-pyridin-4-yl)methyl]-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.60 (Reversed Phase DC-Fertigplatte (E. Merck), Acetonitril/Wasser/ Trifluoressigsäure = 50:50:1)
   Massenspektrum (ESI⁺): m/z = 433 [M+H]⁺
(79) 1-[(5-Cyano-pyridin-2-yl)methyl]-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.60 (Reversed Phase DC-Fertigplatte (E. Merck), Acetonitril/Wasser/ Trifluoressigsäure = 50:50:1)
   Massenspektrum (ESI⁺): m/z = 433 [M+H]⁺
(80) 1-[(Pyrimidin-2-yl)methyl]-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.60 (Reversed Phase DC-Fertigplatte (E. Merck), Acetonitril/Wasser/ Trifluoressigsäure = 50:50:1)
   Massenspektrum (ESI⁺): m/z = 409 [M+H]⁺
(81) 1-[(4-Methyl-pyrimidin-2-yl)methyl]-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.65 (Reversed Phase DC-Fertigplatte (E. Merck), Acetonitril/Wasser/ Trifluoressigsäure = 50:50:1)
   Massenspektrum (ESI⁺): m/z = 423 [M+H]⁺
(82) 1-[(4,6-Dimethyl-pyrimidin-2-yl)methyl]-3-methyl-7-(2-butin-1-yl)-8-(*(*R)-3-amino-piperidin-1-yl)-xanthin
   (BOC-Spaltung erfolgt mit Trifluoressigsäure)
   Schmelzpunkt: 202-204°C
   Massenspektrum (ESI⁺): m/z = 437 [M+H]⁺
(83) 1-[(Chinoxalin-6-yl)methyl]-3-methyl-7-(2-butin-1-yl)-8-((*R*)-3-amino-piperidin-1-yl)-xanthin
   R_{f}-Wert: 0.50 (Kieselgel, Methylenchlorid/Methanol/konz. wässriges Ammoniak = 90:10:0.1)
   Massenspektrum (ESI⁺): m/z = 459 [M+H]⁺
(84) 1-(3-Fluor-4-methoxy-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.45 (Kieselgel, Methylenchlorid/Methanol/konz. wässriges Ammoniak = 90:10:0.1)
   Massenspektrum (ESI⁺): m/z = 455 [M+H]⁺
(85) 1-(3,4-Difluor-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.45 (Kieselgel, Methylenchlorid/Methanol/konz. wässriges Ammoniak = 90:10:0.1)
   Massenspektrum (ESI⁺): m/z = 443 [M+H]⁺
(86) 1-(2-Fluor-5-methoxy-benzyl)-3-methyl-7-(2-butin-1-yl)-8-((*R*)-3-amino-piperidin-1-yl)-xanthin
   R_{f}-Wert: 0.39 (Kieselgel, Methylenchlorid/Methanol/konz. wässriges Ammoniak = 90:10:1)
   Massenspektrum (ESI⁺): m/z = 455 [M+H]⁺
(87) 1-(2-Fluor-3-methoxy-benzyl)-3-methyl-7-(2-butin-1-yl)-8-((*R*)-3-amino-piperidin-1-yl)-xanthin
   R_{f}-Wert: 0.41 (Kieselgel, Methylenchlorid/Methanol/konz. wässriges Ammoniak = 90:10:1)
   Massenspektrum (ESI⁺): m/z = 455 [M+H]⁺
(88) 1-[(4-Cyano-isochinolin-3-yl)methyl]-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.40 (Kieselgel, Methylenchlorid/Methanol/konz. wässriges Ammoniak = 90:10:0.1)
   Massenspektrum (ESI⁺): m/z = 483 [M+H]⁺
(89) 1-(2-Fluor-4-methoxy-benzyl)-3-methyl-7-(2-butin-1-yl)-8-((*R*)-3-amino-piperidin-1-yl)-xanthin
   R_{f}-Wert: 0.45 (Kieselgel, Methylenchlorid/Methanol/konz. wässriges Ammoniak = 90:10:1)
   Massenspektrum (ESI⁺): m/z = 455 [M+H]⁺
(90) 1-[(Furan-2-yl)methyl]-3-methyl-7-(2-butin-1-yl)-8-((*R*)-3-amino-piperidin-1-yl)-xanthin
   (BOC-Spaltung erfolgt mit Trifluoressigsäure)
   Massenspektrum (ESI⁺): m/z = 397 [M+H]⁺
(91) 1-(3,4-Dicyano-benzyl)-3-methyl-7-(2-butin-1-yl)-8-((*R*)-3-amino-piperidin-1-yl)-xanthin x Trifluoressigsäure
   (BOC-Spaltung erfolgt mit Trifluoressigsäure)
   Massenspektrum (ESI⁺): m/z = 457 [M+H]⁺
(92) 1-[(Furan-3-yl)methyl]-3-methyl-7-(2-butin-1-yl)-8-((*R*)-3-amino-piperidin-1-yl)-xanthin
   (BOC-Spaltung erfolgt mit Trifluoressigsäure)
   Massenspektrum (ESI⁺): m/z = 397 [M+H]⁺
(93) 1-[(5-Methyl-furan-2-yl)methyl]-3-methyl-7-(2-butin-1-yl)-8-((*R*)-3-amino-piperidin-1-yl)-xanthin
   (BOC-Spaltung erfolgt mit Trifluoressigsäure)
   Massenspektrum (ESI⁺): m/z = 411 [M+H]⁺
(94) 1-[(5-Brom-furan-2-yl)methyl]-3-methyl-7-(2-butin-1-yl)-8-((*R*)-3-amino-piperidin-1-yl)-xanthin x Trifluoressigsäure
   (BOC-Spaltung erfolgt mit Trifluoressigsäure)
   Massenspektrum (ESI⁺): m/z = 475, 477 [M+H]⁺
(95) 1-(4-Cyano-2-fluor-benzyl)-3-methyl-7-(2-butin-1-yl)-8-((*R*)-3-amino-piperidin-1-yl)-xanthin
   (BOC-Spaltung erfolgt mit Trifluoressigsäure)
   Massenspektrum (ESI⁺): m/z = 450 [M+H]⁺
(96) 1-(2-Cyano-5-fluor-benzyl)-3-methyl-7-(2-butin-1-yl)-8-((*R*)-3-amino-piperidin-1-yl)-xanthin
   (BOC-Spaltung erfolgt mit Trifluoressigsäure)
   Massenspektrum (ESI⁺): m/z = 450 [M+H]⁺
(97) 1-[(5-Cyano-furan-2-yl)methyl]-3-methyl-7-(2-butin-1-yl)-8-((*R*)-3-amino-piperidin-1-yl)-xanthin x Trifluoressigsäure
   (BOC-Spaltung erfolgt mit Trifluoressigsäure)
   Massenspektrum (ESI⁺): m/z = 422 [M+H]⁺
(98) 1-(2-Cyano-6-fluor-benzyl)-3-methyl-7-(2-butin-1-yl)-8-((*R*)-3-amino-piperidin-1-yl)-xanthin
   (BOC-Spaltung erfolgt mit Trifluoressigsäure)
   Massenspektrum (ESI⁺): m/z = 450 [M+H]⁺
(99) 1-(4-Cyano-3-fluor-benzyl)-3-methyl-7-(2-butin-1-yl)-8-((*R*)-3-amino-piperidin-1-yl)-xanthin
   (BOC-Spaltung erfolgt mit Trifluoressigsäure)
   Massenspektrum (ESI⁺): m/z = 450 [M+H]⁺
(100) 1-(2-Cyano-3-methoxy-benzyl)-3-methyl-7-(2-butin-1-yl)-8-((*R*)-3-amino-piperidin-1-yl)-xanthin
   R_{f}-Wert: 0.50 (Kieselgel, Methylenchlorid/Methanol/konz. wässriges Ammoniak = 90:10:0.1)
   Massenspektrum (ESI⁺): m/z = 462 [M+H]⁺
(101) 1-[(8-Cyano-chinolin-7-yl)methyl]-3-methyl-7-(2-butin-1-yl)-8-((*R*)-3-amino-piperidin-1-yl)-xanthin
   (BOC-Spaltung erfolgt mit Trifluoressigsäure)
   Massenspektrum (ESI⁺): m/z = 483 [M+H]⁺
(102) 1-[(4-Cyano-pyridin-3-yl)methyl]-3-methyl-7-(2-butin-1-yl)-8-((*R*)-3-amino-piperidin-1-yl)-xanthin
   (BOC-Spaltung erfolgt mit Trifluoressigsäure)
   Schmelzpunkt: 166°C
   Massenspektrum (ESI⁺): m/z = 433 [M+H]⁺
(103) 1-[(8-Cyano-chinolin-2-yl)methyl]-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.50 (Kieselgel, Methylenchlorid/Methanol/konz. wässriges Ammoniak = 90:10:0.1)
   Massenspektrum (ESI⁺): m/z = 483 [M+H]⁺
(104) 1-[(1-Methyl-1H-benzotriazol-5-yl)methyl]-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.60 (Reversed Phase DC-Fertigplatte (E. Merck), Acetonitril/Wasser/ Trifluoressigsäure = 50:50:1)
   Massenspektrum (ESI⁺): m/z = 462 [M+H]⁺
(105) 1-[(3-Cyano-pyridin-4-yl)methyl]-3-methyl-7-(2-butin-1-yl)-8-((*R*)-3-amino-piperidin-1-yl)-xanthin x Trifluoressigsäure
   (BOC-Spaltung erfolgt mit Trifluoressigsäure)
   R_{f}-Wert: 0.65 (Reversed Phase DC-Fertigplatte (E. Merck), Acetonitril/Wasser/ Trifluoressigsäure = 50:50:1)
   Massenspektrum (ESI⁺): m/z = 433 [M+H]⁺
(106) 1-[(3-Cyano-pyridin-2-yl)methyl]-3-methyl-7-(2-butin-1-yl)-8-((*S*)-3-amino-piperidin-1-yl)-xanthin
   (BOC-Spaltung erfolgt mit Trifluoressigsäure)
   R_{f}-Wert: 0.60 (Reversed Phase DC-Fertigplatte (E. Merck), Acetonitril/Wasser/ Trifluoressigsäure = 50:50:1)
   Massenspektrum (ESI⁺): m/z = 433 [M+H]⁺
(107) 1-[(4-Cyano-benzo[1,3]dioxol-5-yl)methyl]-3-methyl-7-(2-butin-1-yl)-8-((*R*)-3-amino-piperidin-1-yl)-xanthin
   R_{f}-Wert: 0.45 (Kieselgel, Methylenchlorid/Methanol/konz. wässriges Ammoniak = 90:10:0.1)
   Massenspektrum (ESI⁺): m/z = 476 [M+H]⁺

Analog den vorstehenden Beispielen und anderen literaturbekannten Verfahren können auch folgende Verbindungen erhalten werden:
(1) 1-(2-Cyano-4-fluor-benzyl)-3-methyl-7-(2-butin-1-yl)-8-((R)-3-amino-piperidin-1-yl)-xanthin
(2) 1-(2-Cyano-5-fluor-benzyl)-3-methyl-7-(2-butin-1-yl)-8-((R)-3-amino-piperidin-1-yl)-xanthin
(3) 1-(2-Cyano-6-fluor-benzyl)-3-methyl-7-(2-butin-1-yl)-8-((R)-3-amino-piperidin-1-yl)-xanthin
(4) 1-(3-Cyano-4-fluor-benzyl)-3-methyl-7-(2-butin-1-yl)-8-((R)-3-amino-piperidin-1-yl)-xanthin
(5) 1-(3,5-Dicyano-benzyl)-3-methyl-7-(2-butin-1-yl)-8-((R)-3-amino-piperidin-1-yl)-xanthin
(6) 1-(3,4-Dicyano-benzyl)-3-methyl-7-(2-butin-1-yl)-8-((R)-3-amino-piperidin-1-yl)-xanthin
(7) 1-(3-Nitro-4-cyano-benzyl)-3-methyl-7-(2-butin-1-yl)-8-((R)-3-amino-piperidin-1-yl)-xanthin
(8) 1-(2-Chlor-4-cyano-benzyl)-3-methyl-7-(2-butin-1-yl)-8-((R)-3-amino-piperidin-1-yl)-xanthin
(9) 1-(2-Fluor-4-cyano-benzyl)-3-methyl-7-(2-butin-1-yl)-8-((R)-3-amino-piperidin-1-yl)-xanthin
(10) 1-(2-Trifluormethyl-4-cyano-benzyl)-3-methyl-7-(2-butin-1-yl)-8-((R)-3-amino-piperidin-1-yl)-xanthin
(11) 1-[(5-Cyano-pyridin-2-yl)methyl]-3-methyl-7-(2-butin-1-yl)-8-((R)-3-amino-piperidin-1-yl)-xanthin
(12) 1-[(4-Cyano-pyridin-2-yl)methyl]-3-methyl-7-(2-butin-1-yl)-8-((R)-3-amino-piperidin-1-yl)-xanthin
(13) 1-[(4-Cyano-pyridin-3-yl)methyl]-3-methyl-7-(2-butin-1-yl)-8-((R)-3-amino-piperidin-1-yl)-xanthin
(14) 1-[(3-Cyano-pyridin-4-yl)methyl]-3-methyl-7-(2-butin-1-yl)-8-((R)-3-amino-piperidin-1-yl)-xanthin
(15) 1-[(2-Cyano-pyridin-3-yl)methyl]-3-methyl-7-(2-butin-1-yl)-8-((R)-3-amino-piperidin-1-yl)-xanthin
(16) 1-[(2-Cyano-pyridin-4-yl)methyl]-3-methyl-7-(2-butin-1-yl)-8-((R)-3-amino-piperidin-1-yl)-xanthin
(17) 1-[(5-Cyano-pyridin-3-yl)methyl]-3-methyl-7-(2-butin-1-yl)-8-((R)-3-amino-piperidin-1-yl)-xanthin
(18) 1-[(6-Cyano-pyridin-3-yl)methyl]-3-methyl-7-(2-butin-1-yl)-8-((R)-3-amino-piperidin-1-yl)-xanthin
(19) 1-(2-Cyano-4-methoxy-benzyl)-3-methyl-7-(2-butin-1-yl)-8-((R)-3-amino-piperidin-1-yl)-xanthin
(20) 1-(2-Cyano-5-methoxy-benzyl)-3-methyl-7-(2-butin-1-yl)-8-((R)-3-amino-piperidin-1-yl)-xanthin
(21) 1-[([2,2']Bipyridinyl-6-yl)methyl]-3-methyl-7-(2-butin-1-yl)-8-((R)-3-amino-piperidin-1-yl)-xanthin
(22) 1-[(5-Methoxy-pyridin-2-yl)methyl]-3-methyl-7-(2-butin-1-yl)-8-((R)-3-amino-piperidin-1-yl)-xanthin
(23) 1-[(6-Fluor-pyridin-2-yl)methyl]-3-methyl-7-(2-butin-1-yl)-8-((R)-3-amino-piperidin-1-yl)-xanthin
(24) 1-[(5-Cyano-6-methoxy-pyridin-2-yl)methyl]-3-methyl-7-(2-butin-1-yl)-8-((R)-3-amino-piperidin-1-yl)-xanthin
(25) 1-(2-Methoxy-benzyl)-3-methyl-7-(2-butin-1-yl)-8-((R)-3-amino-piperidin-1-yl)-xanthin
(26) 1-(3-Methoxy-benzyl)-3-methyl-7-(2-butin-1-yl)-8-((R)-3-amino-piperidin-1-yl)-xanthin
(27) 1-(3-Chlor-benzyl)-3-methyl-7-(2-butin-1-yl)-8-((R)-3-amino-piperidin-1-yl)-xanthin
(28) 1-(4-Trifluormethyl-benzyl)-3-methyl-7-(2-butin-1-yl)-8-((R)-3-amino-piperidin-1-yl)-xanthin
(29) 1-(3-Trifluormethyl-benzyl)-3-methyl-7-(2-butin-1-yl)-8-((R)-3-amino-piperidin-1-yl)-xanthin
(30) 1-(2-Trifluormethyl-benzyl)-3-methyl-7-(2-butin-1-yl)-8-((R)-3-amino-piperidin-1-yl)-xanthin
(31) 1-(3,4-Dimethoxy-benzyl)-3-methyl-7-(2-butin-1-yl)-8-((R)-3-amino-piperidin-1-yl)-xanthin
(32) 1-(3,4-Dimethoxy-6-fluor-benzyl)-3-methyl-7-(2-butin-1-yl)-8-((R)-3-amino-piperidin-1-yl)-xanthin
(33) 1-[(Benzo[1,3]dioxol-5-yl)methyl]-3-methyl-7-(2-butin-1-yl)-8-((R)-3-amino-piperidin-1-yl)-xanthin

### Beispiel 2

### Dragées mit 75 mg Wirksubstanz

### 1 Drageekern enthält:

| | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1.5 mg |
| | 230,0 mg |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepreßt.
Kerngewicht: 230 mg
Stempel: 9 mm, gewölbt

Die so hergestellten Drageekerne werden mit einem Film überzogen, der im wesentlichen aus Hydroxypropylmethylcellulose besteht. Die fertigen Filmdragees werden mit Bienenwachs geglänzt.
Drageegewicht: 245 mg.

### Beispiel 3

### Tabletten mit 100 mg Wirksubstanz

### Zusammensetzung:

### 1 Tablette enthält:

| | |
|---|---|
| Wirksubstanz | 100,0 mg |
| Milchzucker | 80,0 mg |
| Maisstärke | 34,0 mg |
| Polyvinylpyrrolidon | 4,0 mg |
| Magnesiumstearat | 2.0 mg |
| | 220,0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.

| | |
|---|---|
| Tablettengewicht: | 220 mg |
| Durchmesser: | 10 mm, biplan mit beidseitiger Facette und einseitiger Teilkerbe. |

### Beispiel 4

### Tabletten mit 150 mg Wirksubstanz

### Zusammensetzung:

### 1 Tablette enthält:

| | |
|---|---|
| Wirksubstanz | 150,0 mg |
| Milchzucker pulv. | 89,0 mg |
| Maisstärke | 40,0 mg |
| Kolloide Kieselgelsäure | 10,0 mg |
| Polyvinylpyrrolidon | 10,0 mg |
| Magnesiumstearat | 1.0 mg |
| | 300,0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen.

Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

### Beispiel 5

### Hartgelatine-Kapseln mit 150 mg Wirksubstanz

### 1 Kapsel enthält:

| | |
|---|---|
| Wirkstoff | 150,0 mg |
| Maisstärke getr. | ca. 180,0 mg |
| Milchzucker pulv. | ca. 87,0 mg |
| Magnesiumstearat | 3.0 mg |
| | ca. 420,0 mg |

### Herstellung:

Der Wirkstoff wird mit den Hilfsstoffen vermengt, durch ein Sieb von 0,75 mm-Maschenweite gegeben und in einem geeigneten Gerät homogen gemischt. Die Endmischung wird in Hartgelatine-Kapseln der Größe 1 abgefüllt.
Kapselfüllung: ca. 320 mg
Kapselhülle: Hartgelatine-Kapsel Größe 1.

### Beispiel 6

### Suppositorien mit 150 mg Wirksubstanz

### 1 Zäpfchen enthält:

| | |
|---|---|
| Wirkstoff | 150,0 mg |
| Polyethylenglykol 1500 | 550,0 mg |
| Polyethylenglykol 6000 | 460,0 mg |
| Polyoxyethylensorbitanmonostearat | 840.0 mg |
| | 2000,0 mg |

### Herstellung:

Nach dem Aufschmelzen der Suppositorienmasse wird der Wirkstoff darin homogen verteilt und die Schmelze in vorgekühlte Formen gegossen.

### Beispiel 7

### Suspension mit 50 mg Wirksubstanz

100 ml Suspension enthalten:

| | |
|---|---|
| Wirkstoff | 1,00 g |
| Carboxymethylcellulose-Na-Salz | 0,10 g |
| p-Hydroxybenzoesäuremethylester | 0,05 g |
| p-Hydroxybenzoesäurepropylester | 0,01 g |
| Rohrzucker | 10,00 g |
| Glycerin | 5,00 g |
| Sorbitlösung 70%ig | 20,00 g |
| Aroma | 0,30 g |
| Wasser dest. | ad 100 ml |

### Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.

5 ml Suspension enthalten 50 mg Wirkstoff.

### Beispiel 8

### Ampullen mit 10 mg Wirksubstanz

### Zusammensetzung:

| | |
|---|---|
| Wirkstoff | 10,0 mg |
| 0,01 n Salzsäure s.q. | |
| Aqua bidest | ad 2,0 ml |

### Herstellung:

Die Wirksubstanz wird in der erforderlichen Menge 0,01 n HCl gelöst, mit Kochsalz isotonisch gestellt, sterilfiltriert und in 2 ml Ampullen abgefüllt.

### Beispiel 9

### Ampullen mit 50 mg Wirksubstanz

Zusammensetzung:

| | |
|---|---|
| Wirkstoff | 50,0 mg |
| 0,01 n Salzsäure s.q. | |
| Aqua bidest | ad 10,0 ml |

### Herstellung:

Die Wirksubstanz wird in der erforderlichen Menge 0,01 n HCl gelöst, mit Kochsalz isotonisch gestellt, sterilfiltriert und in 10 ml Ampullen abgefüllt.

## Patentansprüche

1. Verbindungen der allgemeinen Formel 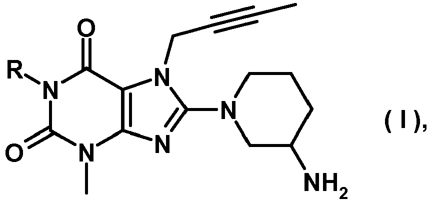 in der
R eine Benzyl-, 2-Fluorbenzyl-, 3-Fluorbenzyl-, 4-Fluorbenzyl-, 2,6-Difluor-benzyl-, 3,4-Difluor-benzyl-, 2-Chlorbenzyl-, 3-Chlorbenzyl- oder 4-Chlorbenzyl-Gruppe, eine 2-Trifluormethyl-benzyl-, 3-Trifluormethyl-benzyl- oder 4-Trifluormethyl-benzyl-Gruppe,
eine 3-Trifluormethoxy-benzyl- oder 4-Trifluormethoxy-benzyl-Gruppe,
eine 2-Cyanobenzyl-, 3-Cyanobenzyl- oder 4-Cyanobenzyl-Gruppe,
eine 2,6-Dicyanobenzyl-, 3,4-Dicyanobenzyl-, 3,5-Dicyanobenzyl-, 2-Trifluormethyl-4-cyano-benzyl-, 3-Nitro-4-cyano-benzyl-, 2-Cyano-3-methoxy-benzyl-, 2-Cyano-4-methoxy-benzyl-, 2-Cyano-5-methoxy-benzyl-, 2-Cyano-4-fluor-benzyl-, 2-Cyano-5-fluor-benzyl-, 2-Cyano-6-fluor-benzyl-, 3-Cyano-4-fluor-benzyl-, 4-Cyano-3-fluorbenzyl-, 2-Fluor-4-cyano-benzyl-, 2-Cyano-3-chlorbenzyl-, 2-Chlor-4-cyano-benzyl-oder 2-Cyano-4-brombenzyl-Gruppe,
eine 2-Methoxy-benzyl-, 3-Methoxy-benzyl-, 4-Methoxy-benzyl-, 2-Fluor-3-methoxy-benzyl-, 2-Fluor-4-methoxy-benzyl-, 2-Fluor-5-methoxy-benzyl-, 3-Fluor-4-methoxy-benzyl-, 3,4-Dimethoxy-benzyl-, 3,5-Dimethoxybenzyl- oder 3,4-Dimethoxy-6-fluorbenzyl-Gruppe,
eine (Benzo[1,3]dioxol-5-yl)methyl-Gruppe,
eine [(4-Cyano-benzo[1,3]dioxol-5-yl)methyl-Gruppe,
eine 2-(3-Cyclopropyloxy-phenyl)-2-oxo-ethyl-, 2-(3-Cyclopropylmethoxy-phenyl)-2-oxo-ethyl- oder 2-(3-Cyclobutyloxy-phenyl)-2-oxo-ethyl-Gruppe,
eine 2-Oxo-2-[2-(pyridin-3-yl)-phenyl]-ethyl- oder 2-Oxo-2-[2-(pyridin-4-yl)-phenyl]-ethyl-Gruppe,
eine (3-Cyano-naphthalin-1-yl)methyl-, (1,4-Dicyano-naphthalin-2-yl)methyl- oder (2,4-Dimethoxy-naphthalin-1-yl)methyl-Gruppe,
eine (Furan-2-yl)methyl-, (Furan-3-yl)methyl-, (5-Brom-furan-2-yl)methyl-, (5-Methyl-furan-2-yl)methyl-, (5-Cyano-furan-2-yl)methyl- oder (5-Methoxycarbonyl-furan-2-yl)methyl-Gruppe,
eine (Pyridin-2-yl)methyl-, (6-Fluor-pyridin-2-yl)methyl- oder (5-Methoxy-pyridin-2-yl)methyl-Gruppe,
eine (3-Cyanopyridin-2-yl)methyl-, (6-Cyanopyridin-2-yl)methyl-, (5-Cyano-pyridin-2-yl)methyl-, (4-Cyano-pyridin-2-yl)methyl-, (4-Cyano-pyridin-3-yl)methyl-, (3-Cyano-pyridin-4-yl)methyl-, (2-Cyano-pyridin-3-yl)methyl-, (2-Cyano-pyridin-4-yl)methyl-, (5-Cyano-pyridin-3-yl)methyl-, (6-Cyano-pyridin-3-yl)methyl- oder (5-Cyano-6-methoxy-pyridin-2-yl)methyl-Gruppe,
eine (6-Phenyl-pyridin-2-yl)methyl- oder eine ([2,2']Bipyridinyl-6-yl)methyl-Gruppe,
eine (Pyrimidin-2-yl)methyl-, (4-Methyl-pyrimidin-2-yl)methyl- oder (4,6-Dimethyl-pyrimidin-2-yl)methyl-Gruppe,
eine (2-Phenyl-pyrimidin-4-yl)methyl- oder (4-Phenyl-pyrimidin-2-yl)methyl-Gruppe,
eine [(1-Methyl-1H-benzotriazol-5-yl)methyl]-Gruppe,
eine (6-Fluor-chinolin-2-yl)methyl-, (7-Fluor-chinolin-2-yl)methyl-, (2-Methyl-chinolin-4-yl)methyl-, (3-Cyano-chinolin-2-yl)methyl-, (3-Cyano-4-methyl-chinolin-2-yl)methyl-, (4-Cyano-chinolin-2-yl)methyl-, (5-Cyano-chinolin-2-yl)methyl-, (8-Cyano-chinolin-2-yl)methyl-, (6-Amino-chinolin-2-yl)methyl-, (8-Amino-chinolin-2-yl)methyl-, (4-Methoxy-chinolin-2-yl)methyl-, (6-Methoxy-chinolin-2-yl)methyl-, (6,7-Dimethoxy-chinolin-2-yl)methyl- oder (8-Cyano-chinolin-7-yl)methyl-Gruppe,
eine (1-Cyano-isochinolin-3-yl)methyl-, (4-Cyano-isochinolin-1-yl)methyl-, (4-Cyano-isochinolin-3-yl)methyl- oder [(4-(Pyridin-2-yl)-isochinolin-1-yl]methyl-Gruppe,
eine (Chinazolin-6-yl)methyl-, (Chinazolin-7-yl)methyl-, (2-Methyl-chinazolin-4-yl)methyl-, (4,5-Dimethyl-chinazolin-2-yl)methyl-, (4-Ethyl-chinazolin-2-yl)methyl-, (4-Cyclopropyl-chinazolin-2-yl)methyl-, (2-Phenyl-chinazolin-4-yl)methyl-, (4-Cyano-chinazolin-2-yl)methyl-, (4-Phenylamino-chinazolin-2-yl)methyl- oder (4-Benzylamino-chinazolin-2-yl)methyl-Gruppe,
eine (Chinoxalin-5-yl)methyl-, (Chinoxalin-6-yl)methyl- oder (2,3-Dimethyl-chinoxalin-6-yl)methyl-Gruppe, oder
eine ([1,5]Naphthyridin-3-yl)methyl-Gruppe bedeutet,
deren Tautomere, Enantiomere, Diastereomere, deren Gemische und deren Salze, zur Verwendung in der Prävention oder Behandlung von Krankheiten oder Zuständen, die in Zusammenhang mit einer erhöhten DPP-IV Aktivität stehen oder durch Hemmung der DPP-IV Aktivität beeinflusst werden können, insbesondere von Diabetes mellitus Typ I oder Typ II oder einer diabetischen Komplikation, wobei die besagten Verbindungen der Formel (I), deren Tautomere, Enantiomere, Diastereomere, deren Gemische oder deren Salze, gegebenenfalls in Kombination mit anderen Wirkstoffen verwendet werden.

2. Eine Verbindung nach Anspruch 1 der allgemeinen Formel 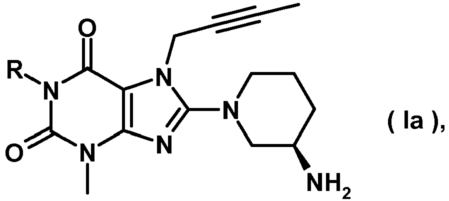 in der R wie in Anspruch 1 erwähnt definiert ist, sowie deren Tautomere und Salze, zur Verwendung in der Prävention oder Behandlung von Krankheiten oder Zuständen, die in Zusammenhang mit einer erhöhten DPP-IV Aktivität stehen oder durch eine Hemmung der DPP-IV Aktivität beeinflusst werden können, insbesondere zur Verwendung in der Behandlung von Diabetes mellitus Typ II oder einer diabetischen Komplikation, wobei die besagte Verbindung der Formel (Ia), deren Tautomere oder Salze, gegebenenfalls in Kombination mit einem anderen Wirkstoff verwendet wird.

3. Eine Verbindung nach Anspruch 1 der allgemeinen Formel 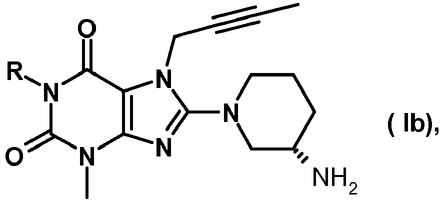 in der R wie in Anspruch 1 erwähnt definiert ist, sowie deren Tautomere und Salze, zur Verwendung in der Prävention oder Behandlung von Krankheiten oder Zuständen, die in Zusammenhang mit einer erhöhten DPP-IV Aktivität stehen oder durch eine Hemmung der DPP-IV Aktivität beeinflusst werden können, insbesondere zur Verwendung in der Behandlung von Diabetes mellitus Typ II oder einer diabetischen Komplikation, wobei die besagte Verbindung der Formel (Ib), deren Tautomere oder Salze, gegebenenfalls in Kombination mit einem anderen Wirkstoff verwendet wird.

4. Physiologisch verträgliche Salze der Verbindungen nach einem der Ansprüche 1 bis 3 mit anorganischen oder organischen Säuren, zur Verwendung in der Prävention oder Behandlung von Krankheiten oder Zuständen, die in Zusammenhang mit einer erhöhten DPP-IV Aktivität stehen oder durch Reduktion der DPP-IV Aktivität verhindert oder gemildert werden können, insbesondere von Diabetes mellitus Typ I oder Typ II oder einer diabetischen Komplikation, wobei die besagten Salze in Kombination mit anderen Wirkstoffen verwendet werden können.

5. Arzneimittel, enthaltend eine Verbindung nach einem der Ansprüche 1 bis 3 oder ein physiologisch verträgliches Salz gemäß Anspruch 4 neben gegebenenfalls einem oder mehreren inerten Trägerstoffen und/oder Verdünnungsmitteln, zur Verwendung in der Prävention oder Behandlung von Krankheiten oder Zuständen, die in Zusammenhang mit einer erhöhten DPP-IV Aktivität stehen oder durch Reduktion der DPP-IV Aktivität verhindert oder gemildert werden können, insbesondere von Diabetes mellitus Typ I oder Typ II oder einer diabetischen Komplikation, wobei die besagte Verbindung nach einem der Ansprüche 1 bis 3 oder das besagte physiologisch verträgliches Salz gemäß Anspruch 4 in Kombination mit einem anderen Wirkstoff verwendet werden kann.

6. Verwendung einer Verbindung nach mindestens einem der Ansprüche 1 bis 4 zur Herstellung eines Arzneimittels, das zur Behandlung von Diabetes mellitus Typ I oder Typ II, einer diabetischen Komplikation, Arthritis, Adipositas, Allograft Transplantation oder durch Calcitonin verursachte Osteoporose geeignet ist, wobei die besagte Verbindung nach mindestens einem der Ansprüche 1 bis 4 gegebenenfalls in Kombination mit einem anderen Wirkstoff verwendet wird.

7. Eine Verbindung nach Anspruch 1 oder 2 der allgemeinen Formel 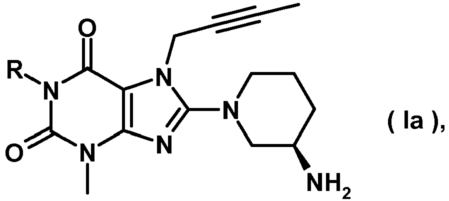 in der R eine (3-Cyanopyridin-2-yl)methyl-, (3-Cyano-chinolin-2-yl)methyl-, 2-Cyanobenzyl-, (4-Methyl-pyrimidin-2-yl)methyl-, (4,6-Dimethyl-pyrimidin-2-yl)methyl-oder (Chinoxalin-6-yl)methyl-Gruppe bedeutet, oder deren physiologisch verträgliches Salz mit einer anorganischen oder organischen Säure, zur Verwendung in der Prävention oder Behandlung von Krankheiten oder Zuständen, die in Zusammenhang mit einer erhöhten DPP-IV Aktivität stehen oder durch Hemmung der DPP-IV Aktivität beeinflusst werden können, insbesondere von Diabetes mellitus Typ I oder Typ II oder einer diabetischen Komplikation, wobei die besagte Verbindung der Formel (Ia) oder deren physiologisch verträgliches Salz, in Kombination mit einem anderen Wirkstoff, z.B. einem Antidiabetikum, verwendet werden kann.

8. Arzneimittel nach Anspruch 5 enthaltend eine Verbindung der allgemeinen Formel 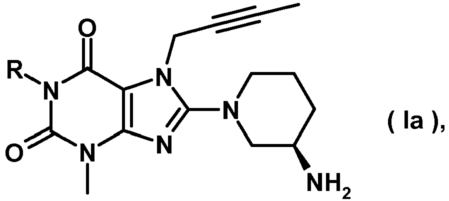 in der R eine (3-Cyanopyridin-2-yl)methyl-, (3-Cyano-chinolin-2-yl)methyl-, 2-Cyanobenzyl-, (4-Methyl-pyrimidin-2-yl)methyl-, (4,6-Dimethyl-pyrimidin-2-yl)methyl-oder (Chinoxalin-6-yl)methyl-Gruppe bedeutet, oder deren physiologisch verträgliches Salz mit einer anorganischen oder organischen Säure, neben gegebenenfalls einem oder mehreren inerten Trägerstoffen und/oder Verdünnungsmitteln, zur Verwendung in der Prävention oder Behandlung von Krankheiten oder Zuständen, die in Zusammenhang mit einer erhöhten DPP-IV Aktivität stehen oder durch Hemmung der DPP-IV Aktivität beeinflusst werden können, insbesondere von Diabetes mellitus Typ I oder Typ II oder einer diabetischen Komplikation, wobei die besagte Verbindung der Formel (Ia) oder deren physiologisch verträgliches Salz, in Kombination mit einem anderen Wirkstoff, z.B. einem Antidiabetikum, verwendet werden kann.

9. Verwendung einer Verbindung der allgemeinen Formel 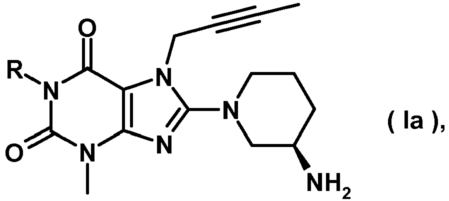 in der R eine (3-Cyanopyridin-2-yl)methyl-, (3-Cyano-chinolin-2-yl)methyl-, 2-Cyanobenzyl-, (4-Methyl-pyrimidin-2-yl)methyl-, (4,6-Dimethyl-pyrimidin-2-yl)methyl-oder (Chinoxalin-6-yl)methyl-Gruppe bedeutet, oder deren physiologisch verträgliches Salz mit einer anorganischen oder organischen Säure, zur Herstellung eines Arzneimittels, das zur Behandlung von Diabetes mellitus Typ I oder Typ II oder einer diabetischen Komplikation geeignet ist, wobei die besagte Verbindung der Formel (Ia) oder deren physiologisch verträgliches Salz gegebenenfalls in Kombination mit einem anderen Wirkstoff, z.B. einem Antidiabetikum, verwendet wird.

10. Die Verbindung
1-[(3-Cyano-pyridin-2-yi)methyl]-3-methyl-7-(2-butin-1-yl)-8-((*R*)-3-amino-piperidin-1-yl)-xanthin,
1-[(3-Cyano-chinoiin-2-yl)methyl]-3-methyl-7-(2-butin-1-yl)-8-((*R*)-3-amino-piperidin-1-yl)-xanthin,
1-(2-Cyano-benzyl)-3-methyl-7-(2-butin-1-yl)-8-((*R*)-3-amino-piperidin-1-yl)-xanthin,
1-[(4-Methyl-pyrimidin-2-yl)methyl]-3-methyl-7-(2-butin-1-yl)-8-((*R*)-3-amino-piperidin-1-yl)-xanthin,
1-[(4,6-Dimethyl-pyrimidin-2-yl)methyl]-3-methyl-7-(2-butin-1-yl)-8-((*R*)-3-amino-piperidin-1-yl)-xanthin, oder
1-[(Chinoxalin-6-yl)methyl]-3-methyl-7-(2-butin-1-yl)-8-((*R*)-3-amino-piperidin-1-yl)-xanthin,
nach Anspruch 1, 2 oder 7 in Form eines physiologisch verträglichen Salzes mit einer anorganischen oder organischen Säure, zur Verwendung in der Prävention oder Behandlung von Krankheiten oder Zuständen, die in Zusammenhang mit einer erhöhten DPP-IV Aktivität stehen oder durch Hemmung der DPP-IV Aktivität beeinflusst werden können, insbesondere von Diabetes mellitus Typ I oder Typ II oder einer diabetischen Komplikation, wobei das besagte Salz in Kombination mit einem anderen Wirkstoff, z.B. einem Antidiabetikum, verwendet werden kann.

11. Arzneimittel nach Anspruch 5 oder 8 enthaltend die Verbindung
1-[(3-Cyano-pyridin-2-y!)methy!]-3-methy!-7-(2-butin-1-y!)-8-((R)-3-amino-piperidin-1-yl)-xanthin,
1-[(3-Cyano-chinolin-2-yl)methyl]-3-methyl-7-(2-butin-1-yl)-8-((*R*)-3-amino-piperidin-1-yl)-xanthin,
1-(2-Cyano-benzyl)-3-methyl-7-(2-butin-1-yl)-8-((*R*)-3-amino-piperidin-1-yl)-xanthin,
1-[(4-Methyl-pyrimidin-2-yl)methyl]-3-methyl-7-(2-butin-1-yl)-8-((*R*)-3-amino-piperidin-1-yl)-xanthin,
1-[(4,6-Dimethyl-pyrimidin-2-yl)methyl]-3-methyl-7-(2-butin-1-yl)-8-((*R*)-3-amino-piperidin-1-yl)-xanthin, oder
1-[(Chinoxalin-6-yl)methyl]-3-methyl-7-(2-butin-1-yl)-8-((*R*)-3-amino-piperidin-1-yl)-xanthin,
in Form eines physiologisch verträglichen Salzes mit einer anorganischen oder organischen Säure, neben gegebenenfalls einem oder mehreren inerten Trägerstoffen und/oder Verdünnungsmitteln, zur Verwendung in der Prävention oder Behandlung von Krankheiten oder Zuständen, die in Zusammenhang mit einer erhöhten DPP-IV Aktivität stehen oder durch Hemmung der DPP-IV Aktivität beeinflusst werden können, insbesondere von Diabetes mellitus Typ I oder Typ II oder einer diabetischen Komplikation, wobei das besagte Salz in Kombination mit einem anderen Wirkstoff, z.B. einem Antidiabetikum, verwendet werden kann.

12. Verwendung der Verbindung
1-[(3-Cyano-pyridin-2-yl)methyl]-3-methyl-7-(2-butin-1-yl)-8-((*R*)-3-amino-piperidin-1-yl)-xanthin,
1-[(3-Cyano-chinolin-2-yl)methyl]-3-methyl-7-(2-butin-1-yl)-8-((*R*)-3-amino-piperidin-1-yl)-xanthin,
1-(2-Cyano-benzyl)-3-methyl-7-(2-butin-1-yl)-8-((R)-3-amino-piperidin-1-yl)-xanthin,
1-[(4-Methyl-pyrimidin-2-yl)methyl]-3-methyl-7-(2-butin-1-yl)-8-((*R*)-3-amino-piperidin-1-yl)-xanthin,
1-[(4,6-Dimethyl-pyrimidin-2-yl)methyl]-3-methyl-7-(2-butin-1-yl)-8-((*R*)-3-amino-piperidin-1-yl)-xanthin, oder
1-[(Chinoxalin-6-yl)methyl]-3-methyl-7-(2-butin-1-yl)-8-((*R*)-3-amino-piperidin-1-yl)-xanthin,
in Form ihres physiologisch verträglichen Salzes mit einer anorganischen oder organischen Säure, zur Herstellung eines Arzneimittels, das zur Behandlung von Diabetes mellitus Typ I oder Typ II oder einer diabetischen Komplikation geeignet ist, wobei das besagte Salz gegebenenfalls in Kombination mit einem anderen Wirkstoff, z.B. einem Antidiabetikum, verwendet wird.

13. Die Verbindungen, Arzneimittel oder Verwendungen nach mindestens einem der vorangehenden Ansprüche, wobei die besagten anderen Wirkstoffe Antidiabetika sind.

14. Die Verbindungen, Arzneimittel oder Verwendungen nach Anspruch 13, wobei die besagten Antidiabetika Metformin, Sulfonylharnstoffe, Nateglinide, Repaglinide, Thiazolidindione, PPAR-gamma Agonisten, alpha-Glucosidasehemmer, Insulin oder Insulinanaloga, oder GLP-1 oder GLP-1 Analoga sind.

## Claims

1. Compounds of general formula 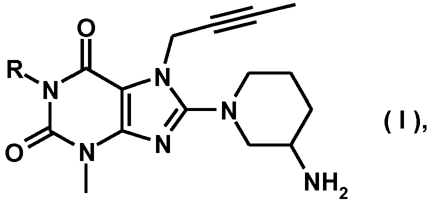 in which
R denotes a benzyl, 2-fluorobenzyl, 3-fluorobenzyl, 4-fluorobenzyl, 2,6-difluoro-benzyl, 3,4-difluoro-benzyl, 2-chlorobenzyl, 3-chlorobenzyl or 4-chlorobenzyl group,
a 2-trifluoromethyl-benzyl, 3-trifluoromethyl-benzyl or 4-trifluoromethyl-benzyl group,
a 3-trifluoromethoxy-benzyl or 4-trifluoromethoxy-benzyl group,
a 2-cyanobenzyl, 3-cyanobenzyl or 4-cyanobenzyl group,
a 2,6-dicyanobenzyl, 3,4-dicyanobenzyl, 3,5-dicyanobenzyl, 2-trifluoromethyl-4-cyanobenzyl, 3-nitro-4-cyano-benzyl, 2-cyano-3-methoxy-benzyl, 2-cyano-4-methoxy-benzyl, 2-cyano-5-methoxy-benzyl, 2-cyano-4-fluoro-benzyl, 2-cyano-5-fluoro-benzyl, 2-cyano-6-fluoro-benzyl, 3-cyano-4-fluoro-benzyl, 4-cyano-3-fluoro-benzyl, 2-fluoro-4-cyano-benzyl, 2-cyano-3-chlorobenzyl, 2-chloro-4-cyano-benzyl or 2-cyano-4-bromobenzyl group,
a 2-methoxy-benzyl, 3-methoxy-benzyl, 4-methoxy-benzyl, 2-fluoro-3-methoxy-benzyl, 2-fluoro-4-methoxy-benzyl, 2-fluoro-5-methoxy-benzyl, 3-fluoro-4-methoxy-benzyl, 3,4-dimethoxy-benzyl, 3,5-dimethoxybenzyl or 3,4-dimethoxy-6-fluoro-benzyl group,
a (benzo[1,3]dioxol-5-yl)methyl group,
a [(4-cyano-benzo[1,3]dioxol-5-yl)methyl group,
a 2-(3-cyclopropyloxy-phenyl)-2-oxo-ethyl, 2-(3-cyclopropylmethoxy-phenyl)-2-oxo-ethyl or 2-(3-cyclobutyloxy-phenyl)-2-oxo-ethyl group,
a 2-oxo-2-[2-(pyridin-3-yl)-phenyl]-ethyl or 2-oxo-2-[2-(pyridin-4-yl)-phenyl]-ethyl group,
a (3-cyano-naphthalen-1-yl)methyl, (1,4-dicyano-naphthalen-2-yl)methyl or (2,4-dimethoxy-naphthalen-1-yl)methyl group,
a (furan-2-yl)methyl, (furan-3-yl)methyl, (5-bromo-furan-2-yl)methyl, (5-methyl-furan-2-yl)methyl, (5-cyano-furan-2-yl)methyl or (5-methoxycarbonyl-furan-2-yl)methyl group,
a (pyridin-2-yl)methyl, (6-fluoro-pyridin-2-yl)methyl or (5-methoxy-pyridin-2-yl)methyl group,
a (3-cyanopyridin-2-yl)methyl, (6-cyanopyridin-2-yl)methyl, (5-cyano-pyridin-2-yl)methyl, (4-cyano-pyridin-2-yl)methyl, (4-cyano-pyridin-3-yl)methyl, (3-cyano-pyridin-4-yl)methyl, (2-cyano-pyridin-3-yl)methyl, (2-cyano-pyridin-4-yl)methyl, (5-cyano-pyridin-3-yl)methyl, (6-cyano-pyridin-3-yl)methyl or (5-cyano-6-methoxy-pyridin-2-yl)methyl group,
a (6-phenyl-pyridin-2-yl)methyl or a ([2,2']bipyridinyl-6-yl)methyl group,
a (pyrimidin-2-yl)methyl, (4-methyl-pyrimidin-2-yl)methyl or (4,6-dimethyl-pyrimidin-2-yl)methyl group,
a (2-phenyl-pyrimidin-4-yl)methyl or (4-phenyl-pyrimidin-2-yl)methyl group,
a [(1-methyl-1H-benzotriazol-5-yl)methyl] group,
a (6-fluoro-quinolin-2-yl)methyl, (7-fluoro-quinolin-2-yl)methyl, (2-methyl-quinolin-4-yl)methyl, (3-cyano-quinolin-2-yl)methyl, (3-cyano-4-methyl-quinolin-2-yl)methyl, (4-cyano-quinolin-2-yl)methyl, (5-cyano-quinolin-2-yl)methyl, (8-cyano-quinolin-2-yl)methyl, (6-amino-quinolin-2-yl)methyl, (8-amino-quinolin-2-yl)methyl, (4-methoxy-quinolin-2-yl)methyl, (6-methoxy-quinolin-2-yl)methyl, (6,7-dimethoxy-quinolin-2-yl)methyl or (8-cyano-quinolin-7-yl)methyl group,
a (1-cyano-isoquinolin-3-yl)methyl, (4-cyano-isoquinolin-1-yl)methyl, (4-cyano-isoquinolin-3-yl)methyl or [(4-(pyridin-2-yl)-isoquinolin-1-yl]methyl group,
a (quinazolin-6-yl)methyl, (quinazolin-7-yl)methyl, (2-methyl-quinazolin-4-yl)methyl, (4,5-dimethyl-quinazolin-2-yl)methyl, (4-ethyl-quinazolin-2-yl)methyl, (4-cyclopropyl-quinazolin-2-yl)methyl, (2-phenyl-quinazolin-4-yl)methyl, (4-cyano-quinazolin-2-yl)methyl, (4-phenylamino-quinazolin-2-yl)methyl or (4-benzylamino-quinazolin-2-yl)methyl group,
a (quinoxalin-5-yl)methyl, (quinoxalin-6-yl)methyl or (2,3-dimethyl-quinoxalin-6-yl)methyl group, or
a ([1,5]naphthyridin-3-yl)methyl group,
the tautomers, enantiomers, diastereomers thereof, the mixtures thereof and the salts thereof, for use in the prevention or treatment of diseases or conditions which are linked to increased DPP-IV activity or can be influenced by inhibiting DPP-IV activity, in particular of type I or type II diabetes mellitus or a diabetic complication, wherein said compounds of formula (I), the tautomers, enantiomers, diastereomers thereof, the mixtures thereof or the salts thereof are optionally used in combination with other active ingredients.

2. A compound according to claim 1 of general formula 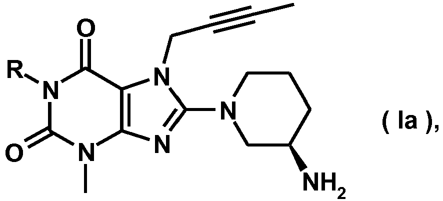 in which R is defined as in claim 1, and the tautomers and salts thereof, for use in the prevention or treatment of diseases or conditions which are linked to increased DPP-IV activity or can be influenced by inhibiting DPP-IV activity, in particular for use in the treatment of type II diabetes mellitus or a diabetic complication, wherein said compound of formula (Ia), the tautomers or the salts thereof is/are optionally used in combination with another active ingredient.

3. A compound according to claim 1 of general formula 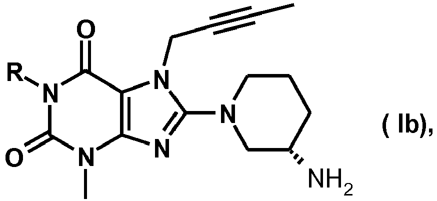 in which R is defined as in claim 1, and the tautomers and salts thereof, for use in the prevention or treatment of diseases or conditions which are linked to increased DPP-IV activity or can be influenced by inhibiting DPP-IV activity, in particular for use in the treatment of type II diabetes mellitus or a diabetic complication, wherein said compound of formula (Ib), the tautomers or the salts thereof is/are optionally used in combination with another active ingredient.

4. Physiologically acceptable salts of the compounds according to any of claims 1 to 3 with inorganic or organic acids, for use in the prevention or treatment of diseases or conditions which are linked to increased DPP-IV activity or can be prevented or alleviated by reducing DPP-IV activity, in particular of type I or type II diabetes mellitus or a diabetic complication, wherein said salts can be used in combination with other active ingredients.

5. Pharmaceutical compositions containing a compound according to any of claims 1 to 3 or a physiologically acceptable salt according to claim 4, optionally together with one or more inert carriers and/or diluents, for use in the prevention or treatment of diseases or conditions which are linked to increased DPP-IV activity or can be prevented or alleviated by reducing DPP-IV activity, in particular of type I or type II diabetes mellitus or a diabetic complication, wherein said compound according to any of claims 1 to 3 or said physiologically acceptable salt according to claim 4 can be used in combination with another active ingredient.

6. Use of a compound according to at least one of claims 1 to 4 for preparing a pharmaceutical composition which is suitable for treating type I or II diabetes mellitus, a diabetic complication, arthritis, obesity, allograft transplantation or calcitonin-induced osteoporosis, wherein said compound according to at least one of claims 1 to 4 is optionally used in combination with another active ingredient.

7. A compound according to either claim 1 or claim 2 of general formula 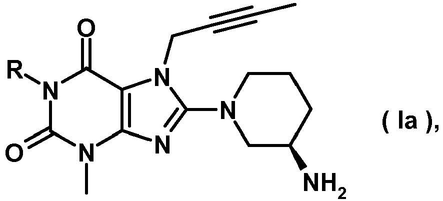 in which R denotes a (3-cyanopyridin-2-yl)methyl, (3-cyano-quinolin-2-yl)methyl, 2-cyanobenzyl, (4-methyl-pyrimidin-2-yl)methyl, (4,6-dimethyl-pyrimidin-2-yl)methyl or (quinoxalin-6-yl)methyl group, or the physiologically acceptable salt thereof with an inorganic or organic acid, for use in the prevention or treatment of diseases or conditions which are linked to increased DPP-IV activity or can be influenced by inhibiting DPP-IV activity, in particular of type I or type II diabetes mellitus or a diabetic complication, wherein said compound of formula (Ia) or the physiologically acceptable salt thereof can be used in combination with another active ingredient, e.g. an anti-diabetic agent.

8. Pharmaceutical compositions according to claim 5, containing a compound of general formula 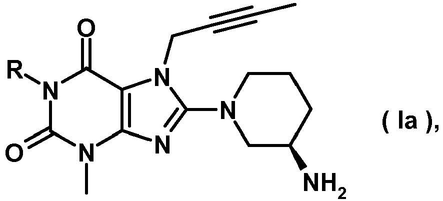 in which R denotes a (3-cyanopyridin-2-yl)methyl, (3-cyano-quinolin-2-yl)methyl, 2-cyanobenzyl, (4-methyl-pyrimidin-2-yl)methyl, (4,6-dimethyl-pyrimidin-2-yl)methyl or (quinoxalin-6-yl)methyl group, or the physiologically acceptable salt thereof with an inorganic or organic acid, optionally together with one or more inert carriers and/or diluents, for use in the prevention or treatment of diseases or conditions which are linked to increased DPP-IV activity or can be influenced by inhibiting DPP-IV activity, in particular of type I or type II diabetes mellitus or a diabetic complication, wherein said compound of formula (Ia) or the physiologically acceptable salt thereof can be used in combination with another active ingredient, e.g. an anti-diabetic agent.

9. Use of a compound of general formula 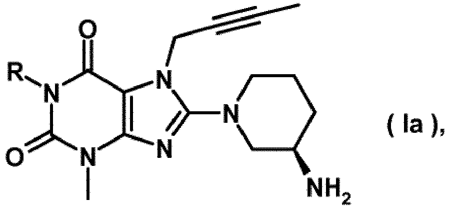 in which R denotes a (3-cyanopyridin-2-yl)methyl, (3-cyano-quinolin-2-yl)methyl, 2-cyanobenzyl, (4-methyl-pyrimidin-2-yl)methyl, (4,6-dimethyl-pyrimidin-2-yl)methyl or (quinoxalin-6-yl)methyl group, or the physiologically acceptable salt thereof with an inorganic or organic acid, for preparing a pharmaceutical composition that is used to treat type I or type II diabetes mellitus or a diabetic complication, wherein said compound of formula (Ia) or the physiologically acceptable salt thereof is optionally used in combination with another active ingredient, e.g. an anti-diabetic agent.

10. The compound
1-[(3-cyano-pyridin-2-yl)methyl]-3-methyl-7-(2-butyn-1-yl)-8-((R)-3-amino-piperidin-1-yl)-xanthine,
1-[(3-cyano-quinolin-2-yl)methyl]-3-methyl-7-(2-butyn-1-yl)-8-((*R*)-3-amino-piperidin-1-yl)-xanthine,
1-(2-cyano-benzyl)-3-methyl-7-(2-butyn-1-yl)-8-((*R*)-3-amino-piperidin-1-yl)-xanthine,
1-[(4-methyl-pyrimidin-2-yl)methyl]-3-methyl-7-(2-butyn-1-yl)-8-((*R*)-3-amino-piperidin-1-yl)-xanthine,
1-[(4,6-dimethyl-pyrimidin-2-yl)methyl]-3-methyl-7-(2-butyn-1-yl)-8-((*R*)-3-amino-piperidin-1-yl)-xanthine, or
1-[(quinoxalin-6-yl)methyl]-3-methyl-7-(2-butyn-1-yl)-8-((*R*)-3-amino-piperidin-1-yl)-xanthine,
according to claim 1, 2 or 7 in the form of a physiologically acceptable salt with an inorganic or organic acid, for use in the prevention or treatment of diseases or conditions which are linked to increased DPP-IV activity or can be influenced by inhibiting DPP-IV activity, in particular of type I or type II diabetes mellitus or a diabetic complication, wherein said salt can be used in combination with another active ingredient, e.g. an anti-diabetic agent.

11. Pharmaceutical compositions according to either claim 5 or claim 8, containing the compound
1-[(3-cyano-pyridin-2-yl)methyl]-3-methyl-7-(2-butyn-1-yl)-8-((*R*)-3-amino-piperidin-1-yl)-xanthine,
1-[(3-cyano-quinolin-2-yl)methyl]-3-methyl-7-(2-butyn-1-yl)-8-((*R*)-3-amino-piperidin-1-yl)-xanthine,
1-(2-cyano-benzyl)-3-methyl-7-(2-butyn-1-yl)-8-((*R*)-3-amino-piperidin-1-yl)-xanthine,
1-[(4-methyl-pyrimidin-2-yl)methyl]-3-methyl-7-(2-butyn-1-yl)-8-((*R*)-3-amino-piperidin-1-yl)-xanthine,
1-[(4,6-dimethyl-pyrimidin-2-yl)methyl]-3-methyl-7-(2-butyn-1-yl)-8-((*R*)-3-amino-piperidin-1-yl)-xanthine, or
1-[(quinoxalin-6-yl)methyl]-3-methyl-7-(2-butyn-1-yl)-8-((*R*)-3-amino-piperidin-1-yl)-xanthine,
in the form of a physiologically acceptable salt with an inorganic or organic acid, optionally together with one or more inert carriers and/or diluents, for use in the prevention or treatment of diseases or conditions which are linked to increased DPP-IV activity or can be influenced by inhibiting DPP-IV activity, in particular of type I or type II diabetes mellitus or a diabetic complication, wherein said salt can be used in combination with another active ingredient, e.g. an anti-diabetic agent.

12. Use of the compound
1-[(3-cyano-pyridin-2-yl)methyl]-3-methyl-7-(2-butyn-1-yl)-8-((R)-3-amino-piperidin-1-yl)-xanthine,
1-[(3-cyano-quinolin-2-yl)methyl]-3-methyl-7-(2-butyn-1-yl)-8-((R)-3-amino-piperidin-1-yl)-xanthine,
1-(2-cyano-benzyl)-3-methyl-7-(2-butyn-1-yl)-8-((*R*)-3-amino-piperidin-1-yl)-xanthine,
1-[(4-methyl-pyrimidin-2-yl)methyl]-3-methyl-7-(2-butyn-1-yl)-8-((*R*)-3-amino-piperidin-1-yl)-xanthine,
1-[(4,6-dimethyl-pyrimidin-2-yl)methyl]-3-methyl-7-(2-butyn-1-yl)-8-((*R*)-3-amino-piperidin-1-yl)-xanthine, or
1-[(quinoxalin-6-yl)methyl]-3-methyl-7-(2-butyn-1-yl)-8-((*R*)-3-amino-piperidin-1-yl)-xanthine,
in the form of the physiologically acceptable salt thereof with an inorganic or organic acid, for preparing a pharmaceutical composition that is suitable for treating type I or type II diabetes mellitus or a diabetic complication, wherein said salt is optionally used in combination with another active ingredient, e.g. an anti-diabetic agent.

13. Compounds, pharmaceutical compositions or uses according to at least one of the preceding claims, wherein said other active ingredients are anti-diabetic agents.

14. Compounds, pharmaceutical compositions or uses according to claim 13, wherein said antidiabetic agents are metformin, sulphonylureas, nateglinides, repaglinides, thiazolidinediones, PPAR-gamma agonists, alpha-glucosidase inhibitors, insulin or insulin analogues, or GLP-1 or GLP-1 analogues.

## Revendications

1. Composés de formule générale 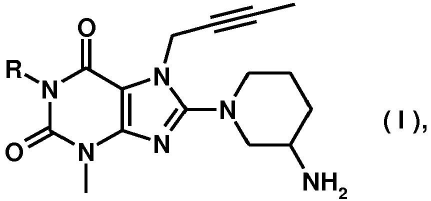 dans laquelle
R représente un groupe benzyle, 2-fluorobenzyle, 3-fluorobenzyle, 4-fluorobenzyle, 2,6-difluorobenzyle, 3,4-difluorobenzyle, 2-chlorobenzyle, 3-chlorobenzyle ou 4-chlorobenzyle,
un groupe 2-trifluorométhyl-benzyle, 3-trifluorométhyl-benzyle ou 4-trifluorométhyl-benzyle,
un groupe 3-trifluorométhoxy-benzyle ou 4-trifluorométhoxy-benzyle,
un groupe 2-cyanobenzyle, 3-cyanobenzyle ou 4-cyanobenzyle,
un groupe 2,6-dicyanobenzyle, 3,4-dicyanobenzyle, 3,5-dicyanobenzyle, 2-trifluorométhyl-4-cyano-benzyle, 3-nitro-4-cyano-benzyle, 2-cyano-3-méthoxy-benzyle, 2-cyano-4-méthoxy-benzyle, 2-cyano-5-méthoxy-benzyle, 2-cyano-4-fluoro-benzyle, 2-cyano-5-fluoro-benzyle, 2-cyano-6-fluoro-benzyle, 3-cyano-4-fluoro-benzyle, 4-cyano-3-fluoro-benzyle, 2-fluoro-4-cyano-benzyle, 2-cyano-3-chlorobenzyle, 2-chloro-4-cyano-benzyle ou 2-cyano-4-bromobenzyle,
un groupe 2-méthoxy-benzyle, 3-méthoxy-benzyle, 4-méthoxy-benzyle, 2-fluoro-3-méthoxy-benzyle, 2-fluoro-4-méthoxy-benzyle, 2-fluoro-5-méthoxy-benzyle, 3-fluoro-4-méthoxy-benzyle, 3,4-diméthoxy-benzyle, 3,5-diméthoxybenzyle ou 3,4-diméthoxy-6-fluoro-benzyle,
un groupe (benzo[1,3]dioxol-5-yl)méthyle,
un groupe [(4-cyano-benzo[1,3]dioxol-5-yl)méthyle,
un groupe 2-(3-cyclopropyloxy-phényl)-2-oxo-éthyle, 2-(3-cyclopropylméthoxy-phényl)-2-oxo-éthyle ou 2-(3-cyclobutyloxy-phényl)-2-oxo-éthyle,
un groupe 2-oxo-2-[2-(pyridin-3-yl)-phényl]-éthyle ou 2-oxo-2-[2-(pyridin-4-yl)-phényl]-éthyle,
un groupe (3-cyano-naphthalèn-1-yl)méthyle, (1,4-dicyano-naphtalèn-2-yl)méthyle ou (2,4-diméthoxy-naphtalèn-1-yl)méthyle,
un groupe (furan-2-yl)méthyle, (furan-3-yl)méthyle, (5-bromo-furan-2-yl)méthyle, (5-méthyl-furan-2-yl)méthyle, (5-cyano-furan-2-yl)méthyle ou (5-méthoxycarbonyl-furan-2-yl)méthyle,
un groupe (pyridin-2-yl)méthyle, (6-fluoro-pyridin-2-yl)méthyle ou (5-méthoxy-pyridin-2-yl)méthyle,
un groupe (3-cyanopyridin-2-yl)méthyle, (6-cyanopyridin-2-yl)méthyle, (5-cyano-pyridin-2-yl)méthyle, (4-cyano-pyridin-2-yl)méthyle, (4-cyano-pyridin-3-yl)méthyle, (3-cyano-pyridin-4-yl)méthyle, (2-cyano-pyridin-3-yl)méthyle, (2-cyano-pyridin-4-yl)méthyle, (5-cyano-pyridin-3-yl)méthyle, (6-cyano-pyridin-3-yl)méthyle ou (5-cyano-6-méthoxy-pyridin-2-yl)méthyle,
un groupe (6-phényl-pyridin-2-yl)méthyle ou ([2,2']bipyridinyl-6-yl)méthyle,
un groupe (pyrimidin-2-yl)méthyle, (4-méthyl-pyrimidin-2-yl)méthyle ou (4,6-diméthyl-pyrimidin-2-yl)méthyle,
un groupe (2-phényl-pyrimidin-4-yl)méthyle ou (4-phényl-pyrimidin-2-yl)méthyle,
un groupe [(1-méthyl-1H-benzotriazol-5-yl)méthyle],
un groupe (6-fluoro-quinolin-2-yl)méthyle, (7-fluoro-quinolin-2-yl)méthyle, (2-méthyl-quinolin-4-yl)méthyle, (3-cyano-quinolin-2-yl)méthyle, (3-cyano-4-méthyl-quinolin-2-yl)méthyle, (4-cyano-quinolin-2-yl)méthyle, (5-cyano-quinolin-2-yl)méthyle, (8-cyano-quinolin-2-yl)méthyle, (6-amino-quinolin-2-yl)méthyle, (8-amino-quinolin-2-yl)méthyle, (4-méthoxy-quinolin-2-yl)méthyle, (6-méthoxy-quinolin-2-yl)méthyle, (6,7-diméthoxy-quinolin-2-yl)méthyle ou (8-cyano-quinolin-7-yl)méthyle,
un groupe (1-cyano-isoquinolin-3-yl)méthyle, (4-cyano-isoquinolin-1-yl)méthyle, (4-cyano-isoquinolin-3-yl)méthyle ou [(4-(pyridin-2-yl)-isoquinolin-1-yl]méthyle,
un groupe (quinazolin-6-yl)méthyle, (quinazolin-7-yl)méthyle, (2-méthyl-quinazolin-4-yl)méthyle, (4,5-diméthyl-quinazolin-2-yl)méthyle, (4-éthyl-quinazolin-2-yl)méthyle, (4-cyclopropyl-quinazolin-2-yl)méthyle, (2-phényl-quinazolin-4-yl)méthyle, (4-cyano-quinazolin-2-yl)méthyle, (4-phénylamino-quinazolin-2-yl)méthyle ou (4-benzylamino-quinazolin-2-yl)méthyle,
un groupe (quinoxalin-5-yl)méthyle, (quinoxalin-6-yl)méthyle ou (2,3-diméthyl-quinoxalin-6-yl)méthyle, ou
un groupe ([1,5]naphtyridin-3-yl)méthyle,
leurs tautomères, énantiomères, diastéréomères, leurs mélanges et leurs sels pour l'utilisation dans la prévention ou le traitement de maladies ou d'états qui sont en relation avec une activité accrue de la DPP-IV ou peuvent être influencés par l'inhibition de l'activité de la DPP-IV, en particulier du diabète sucré de type I ou de type II ou d'une complication diabétique, lesdits composés de formule (I), leurs tautomères, énantiomères, diastéréomères, leurs mélanges ou leurs sels étant utilisés le cas échéant en combinaison avec d'autres substances actives.

2. Composé selon la revendication 1 de formule générale 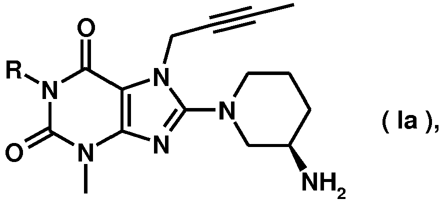 dans laquelle R est tel que défini dans la revendication 1, ainsi que ses tautomères et ses sels, pour l'utilisation dans la prévention ou le traitement de maladies ou d'états qui sont en relation avec une activité accrue de la DPP-IV ou peuvent être influencés par l'inhibition de l'activité de la DPP-IV, en particulier pour l'utilisation dans le traitement du diabète sucré de type II ou d'une complication diabétique, ledit composé de formule (Ia), ses tautomères ou ses sels étant utilisés le cas échéant en combinaison avec une autre substance active.

3. Composé selon la revendication 1 de formule générale 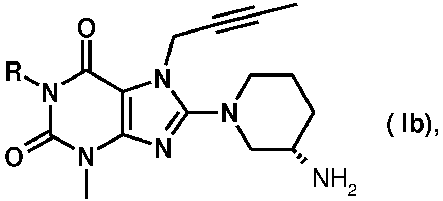 dans laquelle R est tel que défini dans la revendication 1, ainsi que ses tautomères et ses sels, pour l'utilisation dans la prévention ou le traitement de maladies ou d'états qui sont en relation avec une activité accrue de la DPP-IV ou peuvent être influencés par l'inhibition de l'activité de la DPP-IV, en particulier pour l'utilisation dans le traitement du diabète sucré de type II ou d'une complication diabétique, ledit composé de formule (Ib), ses tautomères ou ses sels étant utilisés le cas échéant en combinaison avec une autre substance active.

4. Sels physiologiquement acceptables des composés selon l'une des revendications 1 à 3 avec des acides inorganiques ou organiques, pour l'utilisation dans la prévention ou le traitement de maladies ou d'états qui sont en relation avec une activité accrue de la DPP-IV ou peuvent être inhibés ou soulagés par une réduction de l'activité de la DPP-IV, en particulier du diabète sucré de type I ou de type II ou d'une complication diabétique, lesdits sels pouvant être utilisés en combinaison avec d'autres substances actives.

5. Médicament, contenant un composé selon l'une des revendications 1 à 3 ou un sel physiologiquement acceptable selon la revendication 4, avec le cas échéant un ou plusieurs supports inertes et/ou agents de dilution, pour l'utilisation dans la prévention ou le traitement de maladies ou d'états qui sont en relation avec une activité accrue de la DPP-IV ou peuvent être inhibés ou soulagés par une réduction de l'activité de la DPP-IV, en particulier du diabète sucré de type I ou de type II ou d'une complication diabétique, ledit composé selon l'une des revendications 1 à 3 ou ledit sel physiologiquement acceptable selon la revendication 4 pouvant être utilisé en combinaison avec une autre substance active.

6. Utilisation d'un composé selon au moins l'une des revendications 1 à 4 pour la fabrication d'un médicament qui est approprié au traitement du diabète sucré de type I ou de type II, d'une complication diabétique, de l'arthrite, de l'obésité, de la transplantation d'allogreffe ou de l'ostéoporose provoquée par la calcitonine, ledit composé selon au moins l'une des revendications 1 à 4 étant utilisé le cas échéant en combinaison avec une autre substance active.

7. Composé selon la revendication 1 ou 2 de formule générale 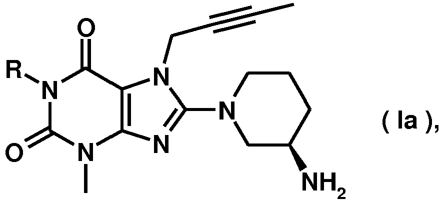 dans laquelle R représente un groupe (3-cyanopyridin-2-yl)méthyle, (3-cyano-quinolin-2-yl)méthyle, 2-cyanobenzyle, (4-méthyl-pyrimidin-2-yl)méthyle, (4,6-diméthyl-pyrimidin-2-yl)méthyle ou (quinoxalin-6-yl)méthyle, ou son sel physiologiquement acceptable avec un acide inorganique ou organique, pour l'utilisation dans la prévention ou le traitement de maladies ou d'états qui sont en relation avec une activité accrue de la DPP-IV ou peuvent être influencés par l'inhibition de l'activité de la DPP-IV, en particulier du diabète sucré de type I ou de type II ou d'une complication diabétique, ledit composé de formule (Ia) ou son sel physiologiquement acceptable pouvant être utilisé en combinaison avec une autre substance active, par exemple un anti-diabétique.

8. Médicament selon la revendication 5, contenant un composé de formule générale 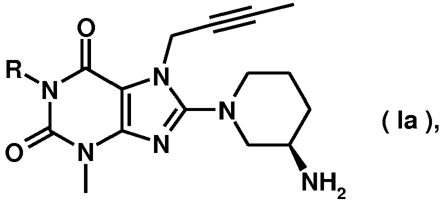 dans laquelle R représente un groupe (3-cyanopyridin-2-yl)méthyle, (3-cyano-quinolin-2-yl)méthyle, 2-cyanobenzyle, (4-méthyl-pyrimidin-2-yl)méthyle, (4,6-diméthyl-pyrimidin-2-yl)méthyle ou (quinoxalin-6-yl)méthyle, ou son sel physiologiquement acceptable avec un acide inorganique ou organique, avec le cas échéant un ou plusieurs supports inertes et/ou agents de dilution, pour l'utilisation dans la prévention ou le traitement de maladies ou d'états qui sont en relation avec une activité accrue de la DPP-IV ou peuvent être influencés par l'inhibition de l'activité de la DPP-IV, en particulier du diabète sucré de type I ou de type II ou d'une complication diabétique, ledit composé de formule (Ia) ou son sel physiologiquement acceptable pouvant être utilisé en combinaison avec une autre substance active, par exemple un anti-diabétique.

9. Utilisation d'un composé de formule générale 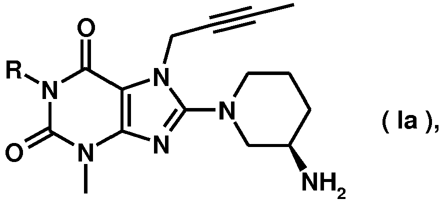 dans laquelle R représente un groupe (3-cyanopyridin-2-yl)méthyle, (3-cyano-quinolin-2-yl)méthyle, 2-cyanobenzyle, (4-méthyl-pyrimidin-2-yl)méthyle, (4,6-diméthyl-pyrimidin-2-yl)méthyle ou (quinoxalin-6-yl)méthyle, ou son sel physiologiquement acceptable avec un acide inorganique ou organique, pour la fabrication d'un médicament qui est approprié au traitement du diabète sucré de type I ou de type II, ou d'une complication diabétique, ledit composé de formule (Ia) ou son sel physiologiquement acceptable pouvant être utilisé le cas échéant en combinaison avec une autre substance active, par exemple un anti-diabétique.

10. Composé
1-[(3-cyano-pyridin-2-yl)méthyl]-3-méthyl-7-(2-butin-1-yl)-8-((*R*)-3-amino-pipéridin-1-yl)-xanthine,
1-[(3-cyano-quinolin-2-yl)méthyl]-3-méthyl-7-(2-butin-1-yl)-8-((*R*)-3-amino-pipéridin-1-yl)-xanthine,
1-(2-cyano-benzyl)-3-méthyl-7-(2-butin-1-yl)-8-((R)-3-amino-pipéridin-1-yl)-xanthine,
1-[(4-méthyl-pyrimidin-2-yl)méthyl]-3-méthyl-7-(2-butin-1-yl)-8-((*R*)-3-amino-pipéridin-1-yl)-xanthine,
1-[(4,6-diméthyl-pyrimidin-2-yl)méthyl]-3-méthyl-7-(2-butin-1-yl)-8-((*R*)-3-amino-pipéridin-1-yl)-xanthine, ou
1-[(quinoxalin-6-yl)méthyl]-3-méthyl-7-(2-butin-1-yl)-8-((R)-3-amino-pipéridin-1-yl)-xanthine,
selon la revendication 1, 2 ou 7, sous la forme d'un sel physiologiquement acceptable avec un acide inorganique ou organique, pour l'utilisation dans la prévention ou le traitement de maladies ou d'états qui sont en relation avec une activité accrue de la DPP-IV ou peuvent être influencés par l'inhibition de l'activité de la DPP-IV, en particulier du diabète sucré de type I ou de type II ou d'une complication diabétique, ledit sel pouvant être utilisé en combinaison avec une autre substance active, par exemple un anti-diabétique.

11. Médicament selon la revendication 5 ou 8 contenant le composé
1-[(3-cyano-pyridin-2-yl)méthyl]-3-méthyl-7-(2-butin-1-yl)-8-((*R*)-3-amino-pipéridin-1-yl)-xanthine,
1-[(3-cyano-quinolin-2-yl)méthyl]-3-méthyl-7-(2-butin-1-yl)-8-((*R*)-3-amino-pipéridin-1-yl)-xanthine,
1-(2-cyano-benzyl)-3-méthyl-7-(2-butin-1-yl)-8-((R)-3-amino-pipéridin-1-yl)-xanthine,
1-[(4-méthyl-pyrimidin-2-yl)méthyl]-3-méthyl-7-(2-butin-1-yl)-8-((*R*)-3-amino-pipéridin-1-yl)-xanthine,
1-[(4,6-diméthyl-pyrimidin-2-yl)méthyl]-3-méthyl-7-(2-butin-1-yl)-8-((*R*)-3-amino-pipéridin-1-yl)-xanthine, ou
1-[(quinoxalin-6-yl)méthyl]-3-méthyl-7-(2-butin-1-yl)-8-((R)-3-amino-pipéridin-1-yl)-xanthine,
sous la forme d'un sel physiologiquement acceptable avec un acide inorganique ou organique, avec le cas échéant un ou plusieurs supports inertes et/ou agents de dilution, pour l'utilisation dans la prévention ou le traitement de maladies ou d'états qui sont en relation avec une activité accrue de la DPP-IV ou peuvent être influencés par l'inhibition de l'activité de la DPP-IV, en particulier du diabète sucré de type I ou de type II ou d'une complication diabétique, ledit sel pouvant être utilisé en combinaison avec une autre substance active, par exemple un anti-diabétique.

12. Utilisation du composé
1-[(3-cyano-pyridin-2-yl)méthyl]-3-méthyl-7-(2-butin-1-yl)-8-((*R*)-3-amino-pipéridin-1-yl)-xanthine,
1-[(3-cyano-quinolin-2-yl)méthyl]-3-méthyl-7-(2-butin-1-yl)-8-((*R*)-3-amino-pipéridin-1-yl)-xanthine,
1-(2-cyano-benzyl)-3-méthyl-7-(2-butin-1-yl)-8-((R)-3-amino-pipéridin-1-yl)-xanthine,
1-[(4-méthyl-pyrimidin-2-yl)méthyl]-3-méthyl-7-(2-butin-1-yl)-8-((*R*)-3-amino-pipéridin-1-yl)-xanthine,
1-[(4,6-diméthyl-pyrimidin-2-yl)méthyl]-3-méthyl-7-(2-butin-1-yl)-8-((*R*)-3-amino-pipéridin-1-yl)-xanthine, ou
1-[(quinoxalin-6-yl)méthyl]-3-méthyl-7-(2-butin-1-yl)-8-((R)-3-amino-pipéridin-1-yl)-xanthine,
sous la forme de son sel physiologiquement acceptable avec un acide inorganique ou organique, pour la fabrication d'un médicament qui est approprié au traitement du diabète sucré de type I ou de type II, ou d'une complication diabétique, ledit sel pouvant être utilisé le cas échéant en combinaison avec une autre substance active, par exemple un anti-diabétique.

13. Composés, médicaments ou utilisations selon au moins l'une des revendications précédentes, lesdites autres substances actives étant des anti-diabétiques.

14. Composés, médicaments ou utilisations selon la revendication 13, lesdits anti-diabétiques étant la metformine, les sulfonylurées, le nateglinide, le répaglinide, la thiazolidinedione, les gamma-agonistes de PPAR, les inhibiteurs de l'alpha-glucosidase, l'insuline ou les analogues de l'insuline ou le GLP-1 ou les analogues du GLP-1.
